(19) Europäisches Patentamt
European Patent Office
Office européen des brevets

(11) EP 2 686 011 B1

(12) **EUROPEAN PATENT SPECIFICATION**

(45) Date of publication and mention of the grant of the patent:
**30.08.2017 Bulletin 2017/35**

(21) Application number: **12725287.2**

(22) Date of filing: **14.03.2012**

(51) Int Cl.:
***A61K 39/12*** *(2006.01)*

(86) International application number:
**PCT/NO2012/050040**

(87) International publication number:
**WO 2012/078051 (14.06.2012 Gazette 2012/24)**

(54) **IPN VACCINE**

IPN-IMPFSTOFF

VACCIN CONTRE LA NÉCROSE PANCRÉATIQUE INFECTIEUSE (IPN)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2011 NO 20110402**
**02.05.2011 NO 20110650**

(43) Date of publication of application:
**22.01.2014 Bulletin 2014/04**

(73) Proprietor: **FVG Limited**
**Inverness IV3 8EX (GB)**

(72) Inventors:
- **EVENSEN, Øystein**
**N-0198 Oslo (NO)**
- **RITCHIE, Gordon**
**N-4085 Hundvåg (NO)**
- **JØSSUND, Trude Bakke**
**N-7745 Oppland (NO)**
- **MUTOLOKI, Stephen**
**N-0673 Oslo (NO)**

(74) Representative: **Mitchell, Simon James et al**
**Urquhart-Dykes & Lord LLP**
**One Euston Square**
**40 Melton Street**
**London NW1 2FD (GB)**

(56) References cited:
**WO-A2-99/50419**

- **H. SONG ET AL: "Molecular Determinants of Infectious Pancreatic Necrosis Virus Virulence and Cell Culture Adaptation", JOURNAL OF VIROLOGY, vol. 79, no. 16, 15 August 2005 (2005-08-15), pages 10289-10299, XP55038140, ISSN: 0022-538X, DOI: 10.1128/JVI.79.16.10289-10299.2005**
- **Orpetveit I, et al.: "Molecular epidemiology of infectious pancreatic necrosis virus (IPNV) in Norway", , 16 October 2008 (2008-10-16), XP007921040, Retrieved from the Internet: URL:http://www.fiskerifond.no/files/projects/attach/sluttrapport_165029_del_2.pdf [retrieved on 2012-09-13]**
- **N M RUANE ET AL: "Molecular differentiation of infectious pancreatic necrosis virus isolates from farmed and wild salmonids in Ireland", JOURNAL OF FISH DISEASES, vol. 32, no. 12, 1 December 2009 (2009-12-01), pages 979-987, XP55038154, ISSN: 0140-7775, DOI: 10.1111/j.1365-2761.2009.01080.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to a live avirulent infectious pancreatic necrosis virus which has been shown to be genetically stable in biological studies. Fish exposed to said virus turn out positive for the virus without showing any signs of disease and the avirulent virus has also been shown to protect the fish against IPN for an extended period of time after administration. Thus, a vaccine comprising said virus and said virus for the prophylaxis or treatment of infectious pancreatic necrosis disease is also part of the present invention.

BACKGROUND OF THE INVENTION

Infectious pancreatic necrosis (IPN) is an economically significant viral disease of salmonid fish worldwide. The disease was first described in freshwater trout in North America in the 1950's and has been reported in Europe since the early 1970's. Initially, IPN was regarded as a serious disease affecting rainbow trout fry and fingerlings. However as the salmon farming industry began to expand during the 1970's, incidence of IPN disease in salmon also increased with the result that IPN is now widespread in the salmon farming industry worldwide. The economic loss due to the disease is large and outbreaks may occur in Atlantic salmon juveniles in fresh-water and in post-smolts after transfer to sea-water.

Infectious pancreatic necrosis virus (IPNV) is the causative agent of IPN and is a member of the Genus *Aquabirnavirus,* family *Birnaviridae.* Aquatic birnaviruses have a wide host range infecting many species of fish. Apart from salmonids they have been isolated from fish belonging to over 32 different families, 11 species of mollusks and four crustacean families.

The IPNV genome consists of two segments of double-stranded RNA that are surrounded by a single-shelled icosahedral capsid of 60 nm in diameter. Genomic segment A (typically 3097 nucleotides) encodes a 106 kDa precursor polyprotein composed of pVP2-VP4-VP3, in that order, and a 15 kDa non-structural VP5 protein, found only in infected cells. Segment B (typically 2777 nucleotides) encodes a minor internal polypeptide VP1 (94 kDa), which is the virion-associated RNA-dependent RNA polymerase (RdRp).

Most aquatic birnaviruses, regardless of host or geographic location, are antigenetically related and belong to a single serogroup A. Serogroup A has been divided into nine serotypes: A1 - A9. The A1 serotype contains most of the isolates from the United States ; serotypes A2 to A5 are primarily European isolates and serotypes A6 to A9 occur in Canada. Serogroup B comprises one serotype isolated from mollusks.

Within the various serotypes/genogroups, there is a high degree of antigenic variability and differences in the virulence and pathogenicity among the strains. Virulence of the IPNV has been associated with segment A and in particular with the VP2 structural protein.

VP2 is a major capsid protein (figure 4) and is responsible for the production of type-specific monoclonal antibodies in hosts which have been infected with the virus. It has been hypothesised that variations in the amino acid residues of this protein may be associated with changes in virulence. In fact, by a comparison of the deduced amino acid sequences of various field isolates exhibiting different mortality in Atlantic salmon fry, the putative motifs involved in virulence of IPNV sp strains have been proposed.

Virulent strains typically have residues threonine, alanine, threonine/alanine, and tyrosine/histidine at positions 217, 221, 247 and 500 of the VP2 sequence. Further work has shown that virulent isolates possess residues Thr217 and Ala221; moderate to low virulent strains have Pro217 and Ala221; and strains containing Thr221 are almost always avirulent, irrespective of the residue at position 217.

Active immunisation of hens using vaccines based on avirulent viruses has long been the industry's standard to protect chickens from an immunosuppressive disease caused by infectious bursal disease virus (IBDV). This virus is a birnavirus like IPNV, but an important difference between IBDV and IPNV is that almost all fish surviving an IPN infection will become long-term carriers of the virus. The IPNV carrier state has no direct negative impact on affected individuals. In persistently infected fish, virus is found associated with leucocytes in blood, head kidney and spleen, presumably in macrophages. Carrier fish shed virus in their faeces, however, titers fluctuate over time and are known to increase in periods of stress.

RNA viruses such as IPNV and IBDV are prone to change through a variety of mechanisms, so it has been assumed that there always is a risk that the virus will revert to greater virulence during multiplication in the vaccinated fish. Such a strategy would require vigilance in monitoring field viruses and the natural history of the disease. So far, unawareness about factors influencing the virulence and genetic stability of IPNV has precluded the use of avirulent strains for vaccine purposes.

An alternative to the use of vaccines based on live avirulent IPNV is inactivated virus vaccines. Killed virus vaccines are prepared by growing virus in large amounts on cell cultures. The virus is then harvested and inactivated with formalin or other similar agents under conditions that ensure the retention of the immunogenic activity of the protective antigens but no virulent virus remains in the vaccine. The current strategies for an inactivated vaccine are expensive, labor intensive and subject to the risk of presence of noninactivated virus. Vaccination with inactivated virus has been tested in rainbow trout given by the oral route, immersion and injection. Protection against challenge was only obtained by injection. However, injection of a large number of fry is not practical in a hatchery situation.

Another option is to use subunit vaccines which are composed of a part of the virus particle responsible for inducing protecting immunity. As previously disclosed, VP2 is a major IPNV capsid protein and is responsible for the production of type-specific monoclonal antibodies. This makes this particular protein a very interesting candidate for use in a subunit vaccine. In fact, vaccination of presmolt with recombinant VP2 (rVP2) included in a commercial oil/glucan-adjuvanted multivalent injectable vaccine resulted in fish protection against IPN in natural outbreaks as compared to fish vaccinated with the same vaccine without the IPN component. This recombinant vaccine against IPNV in Atlantic salmon postsmolts has been approved for commercial use in Norway. However, while older fish can be vaccinated by intraperitoneal injection, this route of administration is not an option in fry due to their small size.

Some producers have also suggested that all parr should be deliberately infected with IPNV as a form of auto-vaccination of survivors, but found that apart from the legal and welfare implications of such a strategy, this would merely transfer the losses from marine to freshwater phase. This is clearly the situation when considering auto-vaccination with a virulent strain.

As it appears from the above summary of the state of the art, there is an industrial need for improved means of controlling IPNV infection in fish, which are non-toxic to the fish and can be administered in an industrially convenient manner and which protect the fish for an extended period of time after administration. Such improvement that are provided by the invention is the treatment of fish to cure or prevent IPNV infection by administering, preferably by immersion, a live avirulent IPNV which is genetically stable in the sense that it does not revert to virulence. Such an avirulent virus has not previously been used, as it hitherto has been considered not an acceptable strategy due to the risk that the avirulent strain revert to virulence.

WO99/50419 describes a system for the generation of live, nonpathogenic infectious pancreatic necrosis virus (IPNV) using synthetic transcripts derived from cloned DNA.

Orpetveit et al., Molecular epidemiology of infectious pancreatic necrosis virus (IPNV) in Norway" (XP007921040) describes a study on the genetic variation of IPNV and associated risk factors for disease outbreaks.

SUMMARY OF THE INVENTION

The present invention is disclosed in the appending claims.

A **first aspect** of the present invention relates to a live avirulent IPNV which do not revert to a virulent virus after at least 3 passages in hosts known to be susceptible to IPNV; said virus comprising a nucleic acid encoding a mature VP2 protein or a precursor VP2 protein comprising an amino acid sequence having at least 85% sequence identity with SEQ ID NO. 1, wherein amino acid residues in position 252, 281, 282 and 319 of the protein are Asn, Ser, Asp and Glu respectively.

One embodiment relates to a live avirulent IPNV according to the first aspect of the present invention, which when delivered by immersion at a titre of 2 x 105 TCID50/ml to Atlantic salmon fry held in fresh water at a temperature of 12 °C causes the fry to be virus positive measured by reisolation on RTG-2 cells.

One embodiment relates to a live avirulent IPNV according to the first aspect of the present invention, which when delivered by immersion at a titre of 2 x 105 TCID50/ml to Atlantic salmon fry held in fresh water at a temperature of 12 °C provides the fry with protection against IPN disease as compared to non-infected fry.

One embodiment relates to a live avirulent IPNV according to the first aspect of the present invention, which when delivered by immersion at a titre of 2 x $10^5$ $TCID_{50}$/ml to Atlantic salmon fry held in fresh water at a temperature of 12 °C does not cause the fry to develop any signs of IPN disease.

In one preferred embodiment, said live avirulent IPNV comprises a nucleic acid encoding a VP2 protein, wherein the amino acid in position 252 of the VP2 protein is not Val.

In one preferred embodiment, said live avirulent IPNV comprises a nucleic acid encoding a VP2 protein, wherein the amino acid in position 252 of the VP2 protein is selected from the group consisting of Ser, Thr, Asn, Gln, Tyr and Cys; preferably selected from the group consisting of Asn and Gln; and most preferably the amino acid residue in position 252 is Asn.

There is described a live avirulent IPNV comprises a nucleic acid encoding a protein comprising an amino acid sequence having at least 80% sequence identity with the amino acid sequence represented by residues 1-442 of SEQIDNO1, with the proviso that the amino acid residue in position 252 of the protein is selected from the group consisting of Ser, Thr, Asn, Gln, Tyr and Cys; preferably selected from the group consisting of Asn and Gln; and most preferably the amino acid residue in position 252 is Asn.

In one preferred embodiment the amino acid residues in position 281, 282 and 319 of the protein are not Thr, Asn and Ala respectively.

In one preferred embodiment the amino acid residues in position 281, 282 and 319 of the protein are Ser, Asp and Glu respectively.

In one preferred embodiment the amino acid residue in position 221 of the protein is Thr.

In one preferred embodiment the amino acid residue in position 217 of the protein is Pro.

In one preferred embodiment said protein causes the avirulent virus not to revert to a virulent virus after at least 3 passages in hosts known to be susceptible to IPNV.

In one particularily preferred embodiment, the live avirulent IPNV according to the first aspect of the present invention is the one designated as IPNV-G700 (deposited under ECACC-No. 11 041201), or closely related strains thereof. Said closely related strains preferably being any strain which shares similar genotypic and/or phenotypic characteristics to the deposited strain.

One preferred embodiment relates to the live avirulent IPNV according to the first aspect of the present invention, which when delivered by immersion at a titre of 2 x $10^5$ $TCID_{50}$/ml to Atlantic salmon fry held in fresh water at a temperature of 12 °C

- causes the fry to be virus positive measured by reisolation on RTG-2 cells;
- causes the fry to be virus negative measured by immunohistochemistry;
- provides the fry with protection against IPN disease as compared to non-infected fry; and
- does not cause the fry to develop any signs of IPN disease.

A **second aspect** of the present invention relates to a vaccine, comprising the live avirulent IPNV according to the first aspect of the present invention.

A **third aspect** of the present invention relates to a live avirulent IPNV according to the first aspect of the present invention, for use as a vaccine in the prophylaxis or treatment of IPN disease.

A **fourth aspect** of the present invention relates to a live avirulent IPNV according to the first aspect of the present invention or a vaccine according to the second aspect of the present invention for use as a vaccine in the prophylaxis or treatment of IPN disease in fry.

A **fifth aspect** of the present invention relates to a live avirulent IPNV according to the first aspect of the present invention or a vaccine according to the second aspect of the present invention for use as a vaccine in the prophylaxis or treatment of IPN disease, wherein distribution is by immersion or oral administration.

Preferred embodiments of the present invention are set forth in the dependent claims and in the detailed description of the invention.

DESCRIPTION OF THE FIGURES

Preferred embodiments of the present invention will now be illustrated in more detail with reference to the accompanying figures.

**Figure 1** illustrates the cumulative mortality of immunized / non-immunized fry challenged / not challenged with a virulent V-1244 strain.

Y-axis

Percent dead fry in each sample (cumulative mortality).

X-axis

| Group | Sample preparation |
|---|---|
| A | Fry immunized with avirulent G700 strain at experimental day 4; challenged with virulent V-1244 strain at experimental day 14; sampling at experimental day 28 |
| B | Fry immunized with avirulent G700 strain at experimental day 4; sampling at experimental day 28 |
| C | Fry immunized with avirulent G700 strain at experimental day 8; challenged with virulent V-1244 strain at experimental day 14; sampling at experimental day 28 |
| D | Fry immunized with avirulent G700 strain at experimental day 8; sampling at experimental day 28 |
| E | Non-immunized fry; challenged with virulent V-1244 strain at experimental day 14; sampling at experimental day 28 |
| F | Non-immunized fry; sampling at experimental day 28 |

(continued)

| Group | Sample preparation |
|---|---|
| G | Fry challenged with avirulent G700 strain at experimental day 14; sampling at experimental day 28 |

**Figure 2** illustrates the cumulative mortality of immunized / non-immunized fry challenged / not challenged with a virulent V-1244 strain.

Y-axis

Percent dead fry in each sample (cumulative mortality).

X-axis

| Group | Sample preparation |
|---|---|
| —— | Fry immunized with avirulent G700 strain at experimental day 4; challenged with virulent V-1244 strain at experimental day 14; sampling at experimental day 28 |
| —◆— | Fry immunized with avirulent G700 strain at experimental day 4; sampling at experimental day 28 |
| —■— | Fry immunized with avirulent G700 strain at experimental day 8; challenged with virulent V-1244 strain at experimental day 14; sampling at experimental day 28 |
| | Fry immunized with avirulent G700 strain at experimental day 8; sampling at experimental day 28 |
| —×— | Non-immunized fry; challenged with virulent V-1244 strain at experimental day 14; sampling at experimental day 28 |
| —✳— | Non-immunized fry; sampling at experimental day 28 |
| —●— | Fry challenged with avirulent G700 strain at experimental day 14; sampling at experimental day 28 |

**Figure 3** illustrates overall number of fry that survived after challenged / not challenged with avirulent G700 strain.

Y-axis

Overall number (percent) of fry that survived after challenged / not challenged with avirulent G700 strain.

X-axis

| | |
|---|---|
| 1 | = unchallenged control |
| 2 | = challenged with avirulent G700 strain |
| 1-3 | = represent data from three different experiments run in parallel |

**Figure 4** illustrates IPNV and the localization of VP1, VP2 and VP3.

**Figure 5** illustrates the amino acid composition at position 217 and 221 of a VP2 protein isolated from an avirulent reverse genetics made strain which has reverted to virulence six months post-infection of fry. In position 217 there is a mixed amino acid composition giving a Pro or Thr amino acid. Combined with position 221 there are representatives of ProThr and ThrAla in the quasispecies pool.

**Figure 6a-b** illustrates aligned sequences of amino acid residues represented as rows within a matrix. Amino acid residues 1-508 and 1-442 represents the amino acid sequence of precursor VP2 protein and mature VP2 protein respectively. The IPNV-VP2 PTA sequence represents the VP2 sequence of an avirulent virus which in example 3 has been shown to revert to a virulent virus after serial passages in fry (instable). The G700 sequence represents the VP2 sequence of an avirulent virus (deposited under ECACC-No. 11 041201) which in example 2 has been shown not to revert to a virulent virus after serial passages in fry (stable). The NVI-015-TA sequence is included as a reference example and represents the VP2 sequence of a virulent virus. Gaps in the identity row indicate positions of non-identical amino acid residues. One and three letter symbols for the amino acids are shown in figure 7a.

**Figure 7a** shows one and three letter symbols for the amino acids.

**Figure 7b** shows the standard genetic code.

## DETAILED DESCRIPTION OF THE INVENTION

An ideal vaccine for IPNV must induce long lasting protection at an early age, prevent virulent carrier formation and be effective against a large number of IPNV serotypes. Injection cannot be used for small fish, therefore either oral delivery or immersion are most preferred routes for early vaccination.

Previously it has been assumed that these attributes of an ideal IPNV vaccine must be met either by a recombinant subunit vaccine or by an inactivated viral vaccine, as a live avirulent vaccine could potentially lead to virulent carrier formation and disease in case the avirulent virus should revert to a virulent virus.

Despite the prevailing technical prejudice within the field, the inventors of the present invention have surprisingly identified an avirulent IPNV strain which induces long lasting protection at an early age, prevents virulent carrier formation, is effective against a large number of IPNV serotypes and may be delivered by oral administration or by immersion, preferably the latter.

The surprising discovery was the result of a project where two different freshwater fish farming sites were followed throughout the production cycle. The sites chosen had either a record of recurrent IPN problems throughout the production cycle, or very few IPN problems, even if the fish were mixed with the IPN-problem group of fish at sea. The fish were followed-up all the way from the brood stock through harvest by frequent samplings and IPNV isolation.

When isolated, the IPNV was subjected to sequencing for detailed characterization at the genomic level. The results demonstrated that both categories of sites harboured IPN virus, but of different genotypes. The site with a history of recurrent IPN problems had a virulent strain while the site with few IPN problems had an avirulent strain, said avirulent strain herein being referred to as the G700 strain (deposited under ECACC-No. 11 041201). Interestingly, the fish infected with the G700 strain seemed to be protected from developing IPN when mixed with fish carrying the virulent strain. These findings revealed a potential for this field strain as a live avirulent vaccine against IPN.

In order to further characterize the virus, serial dilutions of the G700 strain was propagated in CHSE-214 cells with Agarose gel (SeaPlaque) as a solid support (example 2). Three plaques were identified, harvested, purified and cloned. Two of these were subjected to further characterization by sequencing the VP2 gene. The results obtained show that the VP2 sequence of both clones were 100 % identical both at nucleotide and amino acid levels and were also identical to the original G700 strain.

The DNA and RNA sequence of the G700 VP2 gene are presented in SEQ ID NO:2 and SEQ ID NO:3 respectively; and the amino acid sequences of the G700 precursor and mature VP2 protein are represented by amino acid residues 1-508 and 1-442 of SEQ ID NO:1 respectively. As will be seen, both clones had a $Pro_{217}Thr_{221}$ in the virulence motif, consistent with avirulent isolates.

In order to verify that the G700 strain is avirulent and genetically stable in biological studies, Atlantic salmon fry known to be susceptible to IPNV were exposed to the G700 strain at a concentration of 2 x $10^5$ $TCID_{50}$/ml (example 2). The virus was passaged three times before sampling. After sampling, some fry were subjected to stress and then observed for 28 more days before the final sampling. Mortalities were recorded daily.

As can bee seen from figure 3, there was no significant difference between mortalities in the challenged group and the control and no statistical difference was observed between the groups even after subjecting them to stress. Histologically, no pathological lesions were observed in internal organs including the pancreas of the fry before or after stress (data not shown), confirming that the G700 strain in fact is an avirulent strain.

The VP2 sequences of re-isolated viruses were 100 % identical to the original isolate (G700) at the beginning and end of the study, indicating that this variant has a high genetic stability, even after serial passages in fry. In contrast, it has been found that avirulent $Pro_{217}Ala_{221}Ala_{247}$ and avirulent $Pro_{217}Thr_{221}Ala_{247}$ reverse genetics made strains revert to virulent variants over one single infection round of Atlantic salmon fry, thus making the reverse genetics made variants unsuited as candidates for a live avirulent vaccine.

Based on the findings obtained for the G700 strain, a project focused on identifying the amino acid residues responsible for the genetic stability of the avirulent strain was initiated.

By comparing amino acid residues different from the virulence motif (see figure 6a-b), it was surprisingly found that while the instable avirulent strains (easily revert to virulent variants) all have a Val residue in position 252 of the VP2 protein, the G700 strain has an Asn residue in that position. Further, each and all of the instable avirulent strains have a Thr residue in position 281 of the VP2 protein while the G700 strain has a Ser residue in this position. It was also found that while all of the instable avirulent strains have an Asn residue in position 282 and an Ala residue in position 319 of the VP2 protein, the G700 strain has an Asp residue in position 282 and a Glu residue in position 319 (see example 2 and 3).

In summary, the above findings demonstrate that the G700 strain is stable and does not revert to virulence after 3 passages in susceptible hosts. The stability motif of IPN virus has also been identified and consists of residues 252, 281, 282 and 319, of which $Asn_{252}Ser_{281}Asp_{282}Glu_{319}$ is associated with genetic stability (example 2) while $Val_{252}Thr_{281}Asn_{282}Ala_{319}$ is associated with instability (example 3).

A further study was initiated in order to test the protective effect of the G700 strain in Atlantic salmon (*Salmo salar* L.) fry. In addition, the virulence of the field strain was examined by monitoring the mortality of vaccinated (exposed to the G700 strain) but not challenged fry at the stage of their development when they are most susceptible for IPN, to confirm that they do not cause clinical outbreaks of IPN (example 1).

Four days following start-feeding, one group of fish was immunised by immersion with a dosage of 2x $10^5$ $TCID_{50}$/ml of the G700 strain. A second group was vaccinated with the same dosage four days later (8 days) following start feeding. Both vaccinated groups as well as an unvaccinated control group were challenged at day 14 into start feeding, by bath challenge at a dosage of 2x$10^5$ $TCID_{50}$/ml of a virulent strain. In order to examine the mortality as a result of immunization by using the G700 strain, one tank in each group was left unchallenged.

Sampling was performed before vaccination to ensure that all fish were IPN-free at the onset of the study. The second sampling was done at 14 days into start feeding, just before challenge in order to establish whether the carrier status had developed in the vaccinated fish. The sampling was repeated at two and four weeks after challenge. Mortality was recorded on a daily basis.

Fry immunized with the G700 strain were infected and remained so over the duration of the study. The fish did not show any signs of disease before challenge by clinical and laboratory observations (using histology).

Immunization group 1 had slightly better protection against the challenge than immunization group 2, confirmed by mortality rate and histology. The difference in cumulative mortality was about 1, 5% between group 1 and 2, with corresponding relative percent survival (RPS) values of 88 and 82, respectively, confirming a high level of protection.

Histology results from the sampling at 14 days post challenge from group 1 showed 1 fish with pathological presentation out of a total of 6. In group 2, five out of 6 fish had histopathological changes. These results suggest that the earlier the immunization is done after start feeding of the fry, the better the protection will be.

The non-vaccinated groups, challenged with the virulent strain had a cumulative mortality of over 33% on average. The presence of the virus in internal organs and that fish died from IPN was confirmed by immunohistochemistry (data not shown).

In conclusion, the virus strain used as a vaccine (G700 strain) is clearly avirulent since the immunized groups that were not challenge had very low mortality (less than 1, 5% cumulative mortality) and no signs of disease by histology/immunohistochemistry.

Thus, a **first aspect** of the present invention relates to a live avirulent infectious pancreatic necrosis virus (IPNV) which preferably do not revert to a virulent virus after at least 3 passages in hosts known to be susceptible to IPNV.

As used herein, the term "live" as applied to viruses refers to a virus that retains the ability of infecting an appropriate host (as opposed to inactivated or subunit vaccines).

As used herein, the term "avirulent" as applied to viruses is understood to mean a virus strain which has substantially lost, preferably completely lost, its ability to cause disease in fish infected with the strain, although its ability to invade fish, i.e. to penetrate into the fish by the usual route of the virus and to reproduce in the body of the fish, remains substantially intact.

The term "infectious" as applied to viruses indicates that the virus has the ability to reproduce. The virus can be pathogenic or nonpathogenic and still be infectious.

The term "pancreatic necrosis virus (IPNV)" refers to the causative agent of IPN and is a member of the Genus *Aquabirnavirus,* family *Birnaviridae.*

The term "virulent" as applied to viruses herein indicates that the virus is pathogenic, meaning that the virus causes disease to its host.

The term "revert to a virulent virus" as applied to avirulent viruses refers to the process where an avirulent virus revert to a pathogenic virus (meaning that the virus cause disease to its host) by naturally occurring processes (usually a mutation in position 217 and/or 221 of the VP2 protein).

Preferably said virus do not revert to a virulent virus after 3, 5, 10, 15, 20, 25, 30, 35 or 50 passages in hosts known to be susceptible to IPNV. More preferably said virus do not revert to a virulent virus after at least 3, 5, 10, 15, 20, 25, 30, 35 or 50 passages in hosts known to be susceptible to IPNV. Most preferably said virus do not revert to a virulent virus.

In one embodiment according to the first aspect of the present invention, the host is Atlantic salmon *(Salmo salar* L.) fry preferably of the AquaGen breed.

If the live avirulent IPNV according to the first aspect of the present invention is delivered by immersion at a titre of 2 x $10^5$ $TCID_{50}$/ml to Atlantic salmon fry held in fresh water at a temperature of 12 °C it causes the fry to be virus positive measured by reisolation on RTG-2 cells. However, the fry turned out virus negative when measured by immunohistochemistry which suggests that the virus is present in the fry only in subclinical levels.

The term "$TCID_{50}$" as used herein refers to the amount of virus required to produce a cytopathic effect in 50 % of inoculated tissue culture cells. Virus infection in cells is complex and results in many changes to the host cell, known collectively as the cytopathic effect (CPE). Such changes include altered shape, detachment from substrate, lysis, membrane fusion, altered membrane permeability, inclusion bodies and apoptosis. The method used to determine the $TCID_{50}$ value is well known to a man skilled in the art (Beitrag zur kollektiven Behandlung pharmakologischer Reihenversueche, Kärber G., vol. 162, 1931).

Even though the virus was present in the fry only in subclinical levels, the live avirulent IPNV according to the first aspect of the present invention has been shown to provide the fry with protection against IPN disease, in particular as compared to fry which have not been exposed to the live avirulent IPNV according to the first aspect of the present invention.

Further, if the live avirulent IPNV according to the first aspect of the present invention is delivered by immersion at a titre of 2 x $10^5$ $TCID_{50}$/ml to Atlantic salmon fry held in fresh water at a temperature of 12 °C it does not cause the fry to develop any signs of IPN disease, in particular no pathological lesions have been observed in internal organs including the pancreas of the fry (histologically).

As previously disclosed, IPNV has typically two structural proteins which form the IPNV capsid. Virus protein 2 (VP2) of IPNV is one out of these two structural proteins and has been shown to be responsible for the production of type-specific monoclonal antibodies. It has previously been suggested that the sequence of the VP2 protein decides whether the virus is virulent or avirulent, and in case of the latter it has now been shown that certain amino acids of the VP2 protein are important determinants as to whether the avirulent virus may revert to a virulent virus.

Accordingly, in one embodiment according to the first aspect of the present invention the live avirulent IPNV comprises a nucleic acid encoding a mature structural protein (e.g. mature VP2), or a precursor thereof (e.g. precursor VP2), said structural protein preferably causing the avirulent virus not to revert to a virulent virus after at least 3 passages in hosts known to be susceptible to IPNV.

The term "nucleic acid" as used herein refers to a ribonucleic acid (RNA) or a deoxyribonucleic acid (DNA), preferably RNA.

The term "structural protein" as applied to viruses refers to a viral protein that is a structural component (typically a capsid component) of the mature virus.

The term "precursor protein" as used herein refers to a protein which is post- and/or co-translationally modified into its mature form (e.g. precursor VP2 → mature VP2).

Said mature structural protein preferably being one of the two structural proteins which form the IPNV capsid, more preferably the protein forming the outermost part of the IPNV capsid. In one preferred embodiment, said mature structural protein is the protein being responsible for the production of type-specific monoclonal antibodies in hosts which have been infected with the virus. In the most preferred embodiment, said structural protein is the VP2 protein.

Further, as previously discussed it was surprisingly found that while the instable avirulent strains all have a Val residue in position 252 of the VP2 protein, the G700 strain has an Asn residue in that position.

Accordingly, in one embodiment according to the first aspect of the present invention, said virus comprises a nucleic acid encoding a VP2 protein, wherein the amino acid in position 252 of the VP2 protein is not Val.

In another embodiment according to the first aspect of the present invention, said virus comprises a nucleic acid encoding a VP2 protein, wherein the amino acid residue in position 252 of the VP2 protein is selected from the group consisting of Ser, Thr, Asn, Gln, Tyr and Cys; preferably selected from the group consisting of Asn and Gln; and most preferably the amino acid residue in position 252 is Asn.

Further, each and all of the instable avirulent strains have a Thr residue in position 281 of the VP2 protein while the G700 strain has a Ser residue in this position.

Thus, another preferred embodiment according to the present invention relates to a live avirulent IPNV comprising a nucleic acid encoding a mature VP2 and/or precursor VP2 protein, said protein preferably causing the avirulent virus not to revert to a virulent virus after at least 3 passages in hosts known to be susceptible to IPNV; wherein the amino acid in position 281 of the mature VP2 and/or precursor VP2 protein is selected from the group consisting of Ser, Asn, Gln, Tyr and Cys; preferably selected from the group consisting of Ser and Cys; and most preferably the amino acid in position 281 is a Ser residue.

In one embodiment according to the first aspect of the present invention, said virus comprises a nucleic acid encoding a VP2 protein, wherein the amino acid in position 281 of the VP2 protein is not Thr.

It was also found that while all of the instable avirulent strains have an Asn residue in position 282 and an Ala residue in position 319 of the VP2 protein, the G700 strain has an Asp residue in position 282 and a Glu residue in position 319.

Accordingly, another preferred embodiment according to the present invention relates to a live avirulent IPNV comprising a nucleic acid encoding a mature VP2 and/or precursor VP2 protein, said protein preferably causing the avirulent virus not to revert to a virulent virus after at least 3 passages in hosts known to be susceptible to IPNV; wherein the amino acid in position 282 of the mature VP2 and/or precursor VP2 protein is selected from the group consisting of Gln, Asp and Glu; most preferably the amino acid in position 282 of the mature VP2 and/or precursor VP2 protein is Asp.

In one embodiment according to the first aspect of the present invention, said virus comprises a nucleic acid encoding a VP2 protein, wherein the amino acid in position 282 of the VP2 protein is not Asn.

Another preferred embodiment according to the present invention relates to a live avirulent IPNV comprising a nucleic acid encoding a mature VP2 and/or precursor VP2 protein, said protein preferably causing the avirulent virus not to revert to a virulent virus after at least 3 passages in hosts known to be susceptible to IPNV; wherein the amino acid in position 319 of the mature VP2 and/or precursor VP2 protein is selected from the group consisting of Glu and Asp; most preferably the amino acid in position 319 of the mature VP2 and/or precursor VP2 protein is Glu.

In one embodiment according to the first aspect of the present invention, said virus comprises a nucleic acid encoding a VP2 protein, wherein the amino acid in position 319 of the VP2 protein is not Ala.

One particularly preferred embodiment relates to a live avirulent IPNV comprising a nucleic acid encoding a mature VP2 and/or precursor VP2 protein, said protein preferably causing the avirulent virus not to revert to a virulent virus after at least 3 passages in hosts known to be susceptible to IPNV; wherein said mature VP2 and/or precursor VP2 protein has a Pro residue in position 217; a Thr residue in position 221; an Asn residue in position 252; a Ser residue in position 281; an Asp residue in position 282; and a Glu residue in position 319.

The amino acid sequences of the G700 precursor and mature VP2 proteins are represented by amino acid residues 1-508 and 1-442 of SEQ ID NO:1 respectively.

Accordingly, in one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 252-282 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

The term "sequence identity" indicates a quantitative measure of the degree of homology between two amino acid sequences of equal length or between two nucleotide sequences of equal length. The two sequences to be compared must be aligned to best possible fit possible with the insertion of gaps or alternatively, truncation at the ends of the protein sequences. The sequence identity can be calculated as $\frac{(N_{ref}-N_{dif})100}{N_{ref}}$ wherein Ndif is the total number of non-identical residues in the two sequences when aligned and wherein Nref is the number of residues in one of the sequences. A gap is counted as non-identity of the specific residue(s). Sequence identity can alternatively be calculated by the BLAST program e.g. the BLASTP program (Pearson and Lipman 1988) (www.ncbi.nlm.nih.gov/cgi-bin/BLAST).

In one embodiment of the invention, alignment is performed with the sequence alignment method ClustalW with default parameters as described by Thompson J., et al 1994, available at http://www2.ebi.ac.uk/clustalw/.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 252-319 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 200-319 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys;

more preferably Ser or Cys and most preferably Ser; and/or

- preferably with the proviso that residue 282 is selected from the group consisting of Gln, Asp and Glu; most preferably Asp; and/or
- preferably with the proviso that residue 319 is selected from Glu and Asp; most preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 150-319 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 100-319 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 100-350 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; more preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 50-350 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a VP2 protein comprising an amino acid sequence having at least 80% sequence identity with the amino acid sequence represented by residues 50-400 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a protein comprising an amino acid sequence having at least 80% sequence identity with the amino acid sequence represented by residues 1-442 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu; and/or
- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one particularly preferred embodiment according to the first aspect of the present invention, the live avirulent IPNV comprises a nucleic acid encoding a protein comprising an amino acid sequence having at least 80% sequence identity with the amino acid sequence represented by residues 1-508 of SEQIDNO1;

- preferably with the proviso that residue 252 is selected from the group consisting of Ser, Thr, Asn, Gln, Tyr and Cys; more preferably Asn or Gln and most preferably Asn; and/or
- preferably with the proviso that residue 281 is selected from the group consisting of Ser, Asn, Gln, Tyr and Cys; more preferably Ser or Cys and most preferably Ser; and/or
- preferably with the proviso that residue 282 is selected from the group consisting of Gln, Asp and Glu; most preferably Asp; and/or
- preferably with the proviso that residue 319 is selected from Glu and Asp; most preferably Glu; and/or

- preferably with the proviso that residue 217 is Pro; and/or
- preferably with the proviso that residue 221 is Thr;

said protein preferably causing the avirulent virus not to revert to a virulent virus after at least 3 passages in hosts known to be susceptible to IPNV.

In one preferred embodiment according to the first aspect of the present invention, the live avirulent IPNV comprises a nucleic acid encoding a protein comprising or consisting of an amino acid sequence having at least 80% sequence identity with the amino acid sequence represented by residues 1-442 of SEQIDNO1 or the amino acid sequence represented by residues 1-508 of SEQIDNO1; with the proviso that:

- residue 252 is selected from the group consisting of Ser, Thr, Asn, Gln, Tyr and Cys; most preferably Asn;
- residue 281 is selected from the group consisting of Ser, Asn, Gln, Tyr and Cys; most preferably Ser;
- residue 282 is selected from the group consisting of Gln, Asp and Glu; most preferably Asp; and
- residue 221 is Thr.

Further, it is preferred that the amino acid in position 252 is not valine.

As previously discussed, moderate to low virulent strains have $Pro_{217}$ and $Ala_{221}$ in the mature VP2 protein; and strains containing $Thr_{221}$ are almost always avirulent, irrespective of the residue at position 217.

Accordingly, one embodiment according to the first aspect of the present invention relates to a live avirulent infectious pancreatic necrosis virus; said virus comprising a nucleic acid encoding a precursor VP2 and/or a mature VP2 protein; said protein preferably causing the avirulent virus not to revert to a virulent virus after 3 passages in hosts known to be susceptible to IPNV; said precursor VP2 and/or mature VP2 protein preferably having a Pro residue in position 217 and/or a Thr residue in position 221, more preferably at least a Thr residue in position 221.

In one embodiment, the amino acid in position 217 of the mature VP2 and/or precursor VP2 protein is not a Thr residue and/or the amino acid in position 221 of the mature VP2 and/or precursor VP2 protein is not an Ala residue.

In one preferred embodiment according to the present invention, said mature VP2 and/or precursor VP2 protein comprises an amino acid sequence having at least 80% sequence identity with the amino acid sequence represented by residues 200-319 of SEQIDNO1; residues 150-319 of SEQIDNO1; residues 100-319 of SEQIDNO1; residues 100-350 of SEQIDNO1; residues 50-300 of SEQIDNO1; residues 50-350 of SEQIDNO1; residues 50-400 of SEQIDNO1; residues 1-442 of SEQIDNO1; or residues 1-508 of SEQIDNO1 preferably with the proviso that:

- residue 252 is selected from the group consisting of Ser, Thr, Asn, Gln, Tyr and Cys; more preferably Asn or Gln and most preferably Asn; and/or
- residue 281 is selected from the group consisting of Ser, Asn, Gln, Tyr and Cys; more preferably Ser or Cys and most preferably Ser; and/or
- residue 282 is selected from the group consisting of Gln, Asp and Glu; more preferably Asp; and/or
- residue 319 is selected from Glu and Asp; most preferably Glu; and/or
- residue 217 is Pro; and/or
- residue 221 is Thr;

said protein preferably causing the avirulent virus not to revert to virulent virus after at least 3 passages in hosts known to be susceptible to IPNV.

In one preferred embodiment, said sequence identity is selected from the group consisting of at least 85% sequence identity, at least 90% sequence identity, at least 95% sequence identity and 100% sequence identity.

In one particularly preferred embodiment, said mature VP2 and/or precursor VP2 protein comprises an amino acid sequence represented by residue 1-442 of SEQIDNO1.

In another particularly preferred embodiment, said mature VP2 protein consists of an amino acid sequence represented by residue 1-442 of SEQIDNO1 and said precursor VP2 protein consists of an amino acid sequence represented by residue 1-508 of SEQIDNO1.

The precursor VP2 and mature VP2 amino acid sequence of the G700 strain is represented by amino acid residues 1-508 and 1-442 of SEQ ID NO:1 respectively; and provides a convenient reference for the numbering of amino acids as used herein. It is believed that those of skill in the art will readily identify the corresponding positions in other precursor VP2 and mature VP2 proteins.

As previously discussed, genomic segment A of IPNV encodes a 106 kDa precursor polyprotein composed of pVP2-VP4-VP3 in that order. When referring to a nucleic acid molecule encoding a protein, it should be understood that a nucleic acid molecule encoding a polyprotein of which said protein is part of the polyprotein is also meant to be included.

In case the nucleic acid encodes the protein in the form of a polyprotein, it is preferred that the virus also comprises means for separating the polyprotein into separate proteins. Thus, in one embodiment according to the present invention the IPNV virus further comprises a nucleic acid encoding an NS-protease, e.g. a nucleic acid encoding the VP4 protein. Optionally, the means for separating the polyprotein into separate proteins may be present in the host.

Further, in case the nucleic acid encodes a precursor protein (e.g. pVP2) it is preferred that the virus also comprises means for transforming the precursor into its mature form (e.g. mVP2). Optionally, the means for transforming the precursor into its mature form may be present in the host.

A particularily preferred embodiment according to the first aspect of the present invention relates to the G700 strain (IPNV-G700) deposited under ECACC accession No. 11 041201, or closely related strains thereof.
By "closely related strains" we mean any strain which shares similar genotypic and/or phenotypic characteristics to the deposited strain. In particular this phrase encompasses slightly modified forms of the virus which retain substantially the same functional activities. Thus, for example some amino acid or nucleotide additions, deletions or alterations have very little effect; if any, on the functional activities of the virus.

The most preferred embodiment according to the first aspect of the present invention relates to the G700 strain (IPNV-G700) deposited under ECACC accession No. 11 041201.

Another particularily preferred embodiment relates to a live avirulent IPNV according to the first aspect of the present invention, which when delivered by immersion at a titre of 2 x $10^5$ $TCID_{50}$/ml to Atlantic salmon fry held in fresh water at a temperature of 12 °C

- causes the fry to be virus positive measured by reisolation on RTG-2 cells;
- causes the fry to be virus negative measured by immunohistochemistry;
- provides the fry with protection against IPN disease, preferably as compared to non-infected fry; and
- does not cause the fry to develop any signs of IPN disease.

Another particularily preferred embodiment relates to a live avirulent IPNV according to the first aspect of the present invention, which when delivered by immersion at a titre of 2 x $10^5$ $TCID_{50}$/ml to Atlantic salmon fry held in fresh water at a temperature of 12 °C

- causes the fry to be virus positive measured by reisolation on RTG-2 cells;
- causes the fry to be virus negative measured by immunohistochemistry;
- provides the fry with protection against IPN disease, preferably as compared fry which have not been exposed to the live avirulent IPNV according to the first aspect of the present invention; and
- does not cause the fry to develop any signs of IPN disease.

As previously discussed, fish infected with the G700 strain seemed to be protected from developing IPN disease when mixed with fish carrying a virulent strain. These findings revealed a potential for this field strain as a live avirulent vaccine against IPN.

Thus, a second aspect of the present invention relates to a vaccine, comprising the live avirulent IPNV according to the first aspect of the present invention.

A **third aspect** of the present invention relates to the live avirulent IPNV according to the first aspect of the present invention, for use as a vaccine.

In one preferred embodiment according to the third aspect of the present invention, the live avirulent IPNV according to the first aspect of the present invention, is for use as a vaccine against IPN disease in fry and/or smolt and/or fish; more preferably for use as a vaccine against IPN disease in fry.

In one particularily preferred embodiment according to the third aspect of the present invention, the live avirulent IPNV according to the first aspect of the present invention is for vaccination of fry against IPN disease.
In one preferred embodiment according to the third aspect of the present invention, the vaccine is distributed by immersion or oral administration; more preferably distributed to fry by immersion or oral administration.

In another preferred embodiment according to the third aspect of the present invention, said fry are kept in an environment which is free of virulent IPNV for at least 10 days post vaccination with said avirulent IPNV.

In another preferred embodiment according to the third aspect of the present invention, said virus or vaccine is distributed no later than at day 4 after start feeding the fry.

In another preferred embodiment according to the third aspect of the present invention, distribution is by immersion using a virus dosage in the range 1x$10^4$ $TCID_{50}$ / ml to 1 x $10^6$ $TCID_{50}$ / ml; more preferably 5x$10^4$ $TCID_{50}$ / ml to 5 x $10^5$ $TCID_{50}$ / ml; even more preferably 1x$10^5$ $TCID_{50}$ / ml to 5 x $10^5$ $TCID_{50}$ / ml; and most preferably about 2 x $10^5$ $TCID_{50}$ / ml.

The present invention also relates to a live avirulent IPNV according to the first aspect of the present invention; or a vaccine according to the second aspect of the present invention; for the prophylaxis or treatment of IPN disease.

The present invention also relates to a live avirulent IPNV according to the first aspect of the present invention; or vaccine according to the second aspect of the present invention; for the prophylaxis or treatment of IPN, wherein distribution is by injection, immersion or as a food additive, preferably immersion or as a food additive, most preferably by immersion.

The present invention also relates to a live avirulent IPNV according to the first aspect of the present invention; or a vaccine according to the second aspect of the present invention, for the prophylaxis or treatment of IPN disease in fish and/or fry; preferably fry.

The present invention also relates to a live avirulent IPNV according to the first aspect of the present invention; or a vaccine according to the second aspect of the present invention; for the prophylaxis or treatment of IPN disease in fry, wherein said fry are kept in an environment which is free of virulent IPNV for at least 10 days post vaccination with said avirulent IPNV.

The present invention also relates to a live avirulent IPNV according to the first aspect of the present invention; or a vaccine according to the second aspect of the present invention, for the prophylaxis or treatment of IPN disease in fry, wherein said virus or vaccine is administered no later than at day 4 after start feeding the fry.

The present invention also relates to a live avirulent IPNV according to the first aspect of the present invention; or a vaccine according to the second aspect of the present invention, for the prophylaxis or treatment of IPN disease, wherein distribution is by immersion using a virus dosage in the range $1x10^4$ $TCID_{50}$ / ml to 1 $x10^6$ $TCID_{50}$ / ml; more preferably $5x10^4$ $TCID_{50}$ / ml to 5 x $10^5$ $TCID_{50}$ / ml; even more preferably 1 x $10^5$ $TCID_{50}$ / ml to 5 x $10^5$ $TCID_{50}$ / ml; and most preferably about $2x10^5$ $TCID_{50}$ / ml.

Having now fully described the present invention in some detail by way of illustration and example for purpose of clarity of understanding, it will be obvious to one of ordinary skill in the art that same can be performed by modifying or changing the invention by with a wide and equivalent range of conditions, formulations and other parameters thereof, and that such modifications or changes are intended to be encompassed within the scope of the appended claims.

EXAMPLES

The following examples are meant to illustrate how to make and use the invention. They are not intended to limit the scope of the invention in any manner or to any degree.

**Example 1**

**"Effect of IPN-vaccination"**

Vaccine strain

The vaccine strain, herein referred to as the G700 strain, was a field isolate of IPNV collected from one year old fish in sea. The fish from which the virus was isolated was clinically healthy and not suffering from IPN. The vaccine strain was propagated in RTG-2 cells and titrated in CHSE-214 cells. (Titer: 2,0 $x10^8$ $TCID_{50}$/ml, dose per tank: 2,0 ml, diluted to 10 ml using L-15 medium.)

IPNV challenge material

The challenge strain of IPNV was the virulent V-1244 strain. This strain was propagated in RTG-2 cells and titrated in CHSE cells. (Titer: 9,8 $x10^6$ $TCID_{50}$/ml, dose per tank: 20ml.)

Test subs eft

The fish used were wild salmon fry, approximately1560 in total, originating from the river Rauma. The eggs came from brood fish kept at the living gene bank Haukvik, S-Trøndelag for more than 10 years. The eggs were hatched at VESO Vikan's hatchery, and transferred to the research station when ready for start-feeding. The fish were fed according to S-2002.

Environment parameters during experiment

| | |
|---|---|
| Water quality: | Fresh water |
| Temperature: | 12 ± 1 °C |
| Oxygen level: | Minimum 8 mg/L at outlet |
| Flow: | Flow should be kept at a level to ensure sufficient $pO_2$ (0.1 L per tank per minute) |

Experimental procedure

| Task | Activity | Exp. day |
|---|---|---|
| Disinfection | Thirteen tanks, each having a total water volume of 8L, were disinfected according to standard procedures prior to start of experiment. | 0 |
| Begin start feeding | 1560 fry were distributed into the thirteen separate tanks of 120 fish each (group $1_{A-D}$, $2_{A-D}$ and $3_{A-E}$). | 0 |
| Sampling | 20 fry were weighed and then frozen at -20°C | 1 |
| Immunization group 1 | Fish in four parallel tanks $1_{A-D}$) were immunized by pausing the water flow, and adding 2 x $10^5$ $TCID_{50}$/ml of the G700 strain to the water. Normal flow was resumed after three hours. | 4 |
| Immunization group 2 | Fish in four parallel tanks ($2_{A-D}$) were immunized by pausing the water flow, and adding 2 x $10^5$ $TCID_{50}$/ml of the G700 strain to the water. Normal flow was resumed after three hours. | 8 |
| Sampling | Six fish were collected from all parallels in groups 1, 2 and 3, and frozen at -20°C. Six fish from all parallels in groups 1, 2 and 3 were sampled for histopathological examination bv submersion in formalin. | 14 |
| Challenge | Fish in immunization group $1_{A-c}$, immunization group $2_{A-c}$ and non-immunized group $3_{A-c}$ were challenged by pausing the water flow and adding 2x$10^5$ $TCID_{50}$/ml of the V-1244 strain to the water. The flow of water was paused in the control tanks ($1_D$, $2_D$ and $3_{D-E}$) as well, and normal flow was resumed after three hours. | 14 |
| Sampling | Six fish are collected from all parallels in groups 1, 2 and 3, and frozen at -20°C. Six fish from all parallels in groups 1, 2 and 3 were sampled for histopathological examination by submersion in formalin. | 28 |
| Sampling | Six fish are collected from all parallels in groups 1, 2 and 3, and frozen at -20°C. Six fish from all parallels in groups 1, 2 and 3, were sampled for histopathological examination by submersion in formalin. | 42 |
| End of experiment | Any remaining fish were killed and frozen at -70°C. | 43 |

Results, re-isolation of virus by cell culture and RT-PCR

Immunization groups $1_{A-D}$ and $2_{A-D}$ as well as non-immunized groups ($3_{A-E}$) were sampled before challenge at experimental day 14. The immunized groups turned out positive for IPNV after reisolation on RTG-2 cells. This was confirmed with positive RT PCR results. The non-immunized groups were negative on both cell culture and RT PCR (table 1).

The sampling was repeated after challenge at experimental day 28. The results show that all groups that has been either immunized and/or challenged (groups $1_{A-D}$, $2_{A-D}$ and $3_{A-C}$) were positive for the virus. The non-immunized and non-challenged groups ($3_{D-E}$) were negative by cell culture. However, 2 out of 12 samples turned out positive by PCR (table 1).

The last sampling at experimental day 42 shows positive results in immunization group 1 and 2 ($1_{A-C}$ and $2_{A-C}$), but 7 of the samples in group 2 were found positive by PCR. Similarly, 4 out of 18 samples in the non-immunized/challenged groups ($3_{A-C}$) were also found positive by PCR, although not by virus re-isolation. The negative controls ($3_{D-E}$) were all negative on cell culture and RT PCR (table 1).

Table 1

| Group | Tank | Pre-challenge Exp. day 14 | | | Post-challenge Exp. day 28 | | | Post-challenge Exp. day 42 | | |
|---|---|---|---|---|---|---|---|---|---|---|
| | | Total | + | - | Total | + | - | Total | + | - |
| 1 | A, B, C | 18 | 18 | 0 | 18 | 18 | 0 | 18 | 18 | 0 |
| 2 | A, B, C | 18 | 18 | 0 | 18 | 18 | 0 | 18 | 15 | (3) |

(continued)

| Group | Tank | Pre-challenge Exp. day 14 | | | Post-challenge Exp. day 28 | | | Post-challenge Exp. day 42 | | |
|---|---|---|---|---|---|---|---|---|---|---|
| | | Total | + | - | Total | + | - | Total | + | - |
| 3 | A, B, C | 18 | 0 | 18 | 18 | 18 | 0 | 18 | 14 | (4) |
| 1 | D | 6 | 6 | 0 | 6 | 6 | 0 | 6 | 6 | 0 |
| 2 | D | 6 | 6 | 0 | 6 | 6 | 0 | 6 | 2 | (4) |
| 3 | D, E | 12 | 0 | 12 | 12 | 2 | 10 | 12 | 0 | 12 |

*Virus re-isolation results of fry sampled at different time points. Homogenates were tested by cell-culture (RTG-2) inoculation and observation for CPE followed by verification by RT-PCR. Numbers in brackets represent positive results only by RT-PCR (PCR have higher sensitivity than cell culture).*

Results, mortality

Mortality was recorded daily and dead fish (if any) were removed from the tanks daily. Results are presented in figure 1 and figure 2.

**Example 2**

**"Avirulence and genetic stability"**

Plaque purification of the vaccine strain

Plaques of IPNV were obtained by propagating serial dilutions of the G700 strain in CHSE-214 cells, with Agarose gel (SeaPlaque) as a solid support. Three plaques were indentified, harvested, purified and cloned. Two of these were subjected to further characterization by sequencing the VP2 gene.

The results obtained show that the VP2 sequences of both clones were 100% identical both at nucleotide and amino acid levels and were identical to the original G700 strain. The clones had $Pro_{217}Thr_{221}$ in the virulence motif, consistent with low virulent isolates. However, only one the two clones were used in subsequent challenge experiments.

The nucleotide sequence of the G700 VP2 gene is presented in SEQ ID NO:2 and the amino acid sequence of the G700 mature VP2 protein is represented by amino acid residue 1-442 of SEQ ID NO:1.

Avirulence and genetic stability

Challenge of fry was done at Veso-Vikan, an accredited experimental facility meeting the requirements of existing regulations (based on General European Pharmacopoeia monographs for documentation of live virus vaccines). Atlantic salmon fry of the AquaGen breed known to be susceptible to IPNV were used. The fry were exposed to the virus solution (challenged) at the point of start-feeding. Although the initial objective was to passage the virus 6 times in fry, it was only possible to undertake 3 passages owing to the seasonality in the availability of fry. The study ran as discontinuous experimental blocks, one after another, with laboratory work (virus re-isolation and propagation for use in the next experiment) in-between. Each experiment comprised three parallels, 100 fry in each bucket. A fourth bucket containing 100 fry was also included as control against background mortality. The dosage of virus was about $2x10^5$ $TCID_{50}$/ml and was administered by bath at approximately 6 days after start-feeding. The first sampling was done at day 21 following virus exposure. After sampling, the fry were subjected to stress (lowering the water content in the buckets combined with movement of a netpen around in the buckets for a period of 10 min; this procedure repeated daily for 1 week) in the 1$^{st}$ and 3$^{rd}$ experimental blocks. These fry were then observed for 28 more days before the final sampling. Mortalities were recorded daily.

Figure 3 shows the overall mortalities at the end of each experimental block. In the first experiment, there was slightly more background mortalities in both the control and immunized groups both before and after stress. After stress however, 5 more fry died on average in the challenged group compared to the control although ultimately, about 90% of the fry survived and there was no significant difference (p=0.05) between the challenged group and the control. In the second and third experiments, very few fish died in either group with no statistical difference being observed between the groups even after subjecting them to stress (3$^{rd}$ experiment).

Histolologically, no pathological lesions were observed in internal organs including the pancreas of the fry before or after stress. Furthermore, no IPN virus was detected by immunohistochemistry in any of the immunized fry although the virus was re-isolated from them. This suggests that the virus was present in the fry only in subclinical levels. The VP2 sequences of re-isolated viruses were 100% identical to the original isolate (G700) at the beginning and end of the study. In the 2nd and 3rd passage prior to stress however, a mutation from leucine to phenylalanine at position 336 was observed. This is a conserved mutation and has no influence on the virulence of the virus since it falls outside the critical motifs. This is supported by the low mortalities associated with the isolates. It is also noteworthy that the mutation reverted following stress of the fry in the 3rd experiment.

**Example 3**

**"Avirulence and genetic instability"**

Cells and viruses

Chinock salmon embryo (cells (CHSE-214; ATCC CCL-1681) were maintained at 20 °C in L-15 medium (Sigma-Aldrich) supplemented with 5 % fetal bovine serum (FBS, Medprobe), 2 mM L-glutamine (Sigma-Aldrich) and 50 $\mu$g ml$^{-1}$ gentamicin (Sigma-Aldrich). Rainbow trout gonad cells (RTG-2; ATCC CCL-55) were grown at 20 °C in L-15 medium supplemented with 10% fetal bovine serum, 2 mM L-glutamine and 50 $\mu$g ml$^{-1}$ gentamicin.

The virus isolates were propagated in RTG-2 cells by inoculation of 100$\mu$l virus stock solution (stored at -20 °C in 30 % glycerol, titer ~ $10^7$ TCIDso/ml) onto 70 % confluent T-162 cm$^2$ cell culture flasks. The supernatants were harvested when widespread cytopathic effect was visible, 5-7 days post infection. The cell culture supernatants were obtained after a centrifugation at 2500 x g for 10 minutes, and sterile filtration (0.22$\mu$l). The infectious titer was determined by end point dilution on CHSE-214 cells, and the $TCID_{50}$ was estimated by the method of Karber (1931. Beitrage zur kollektiven Behandlung pharmakologischer Reihenversuche. Arch.Exp.Pathol.Pharmakol 162:480-483.).

Construction of full-length cDNA clones

Generation of full-length cDNA clones of the entire coding and non-coding regions of NVI-015 RNA segment A and B was performed according to procedures described by Yao and Vakharia (1998. Generation of infectious pancreatic necrosis virus from cloned cDNA. J Virol. 72:8913-8920). The recombinant IPNV Sp isolate rNVI-15PA (also named rNVI15VP2) was generated as described previously by Song and co-workers (2005. Molecular determinants of infectious pancreatic necrosis virus virulence and cell culture adaptation. Journal of Virology 79:10289-10299). This recombinant isolate originates from rNVI-15TA (also named rNVI15) and the 5'end of Sp103. This resulted in the recovery of the viral progeny with a virulence motif similar to the parent isolate Sp103 $Pro_{217}Ala_{221}Ala_{247}$ (2005. Molecular determinants of infectious pancreatic necrosis virus virulence and cell culture adaptation. Journal of Virology 79:10289-10299). Sp103 is an IPNV isolate obtained from a field outbreak of IPN, and causes moderate to low rates of mortality (2005. Infectious pancreatic necrosis virus induces apoptosis in vitro and in vivo independent of VP5 expression. Virology 342:13-25; 2004. Identification of putative motifs involved in the virulence of infectious pancreatic necrosis virus. Virology 322:31-40). The recombinant rNVI-15PA virus was subjected to two rounds of plaque purification on CHSE-214 cells before propagation in RTG-2 cells to obtain a stock. The nucleic acid of the virus was sequenced completely to ensure that no advert mutation occurred due to cell adaptation.

The genetically engineered virus strains were propagated on RTG-2 monolayers. The supernatants were harvested following centrifugation at 2500 x g for 10 minutes and sterile filtration (0.22$\mu$l). RNA extraction using the QIAamp viral mini kit (Qiagen) was carried out according to the manufacturer's recommendation. To ensure that the generated clones had the correct residues, the genomes of segment A and B were sequenced as described by Yao and Vakharia (1998. Generation of infectious pancreatic necrosis virus from cloned cDNA. J Virol. 72:8913-8920) and the chromatograms were analyzed.

Plaque purification assay

To generate the final mutant virus in residue 221 of VP2 we passaged the obtained ProAlaAla recombinant virus 10 times (passages) in CHSE-214 cells (2005. Molecular determinants of infectious pancreatic necrosis virus virulence and cell culture adaptation. Journal of Virology 79:10289-10299). Then the isolate was plaque purified by inoculating RTG-2 monolayers on six well plates with 10-fold dilution ($10^{-3}$ to $10^{-8}$) of cell culture supernatants. After 1hr adsorption at room temperature the inoculum was removed and the cells were overlaid with 0.8 % SeaPlaque Agarose (BioWhittaker) in 2xL-15 medium (Sigma) containing 5 % FBS and 1 % L-glutamine. The cells were incubated at 15 °C for 4 days and plaques formed by cytopathic effect (CPE) were picked by insertion of a 3mm punch biopsy (Miltex) through the agar to the plate. The plaques were subsequently inoculated on RTG-2 monolayers and incubated at 15 °C until full CPE was observed. Then the supernatant was harvested following a centrifugation at 2500 x g for 10 minutes and sterile filtration (0.22μl). RNA was extracted using the QIAamp Viral RNA mini kit according to the manufacturer's recommendations. Complete nucleotide sequences of segment A and B of each virus was determined as described before. The chromatographs were checked to ensure a "clean" ATT codon encoding $Thr_{221}$ was used in the study. No other mutations were found in the entire genome of the virus compared to rNVI-15PA. Prior to challenge the isolates were propagated by one passage in RTG-2 cells.

Establishing a population of persistently infected Atlantic salmon fry

The challenge was conducted at VESO Vikan's research facility, Namsos, Norway. This was part of a larger study including in total 1020 Atlantic salmon (*Salmo salar* L.) fry of the AquaGen strain hatched at the VESO Vikan hatchery were included in the experiment. The fry had recently started to feed (Micro 015, Ewos) and had an average weight of 0.2 grams (n=20). The fish were divided into 4 buckets, each of 250 fry. After an acclimatization period of one week, the fry were starved one day prior to challenge.

Fish were challenged by immersion with rNVI-15PT at a dose of 5 $x10^4$ $TCID_{50}$/ml in a total volume of 4 liters (Tank 1). One control tank was mock-infected by adding cell culture medium (Tank 2). The water was aerated during the challenge. After a period of 3 hours the water volume was reduced to 2 liters and normal flow was resumed. Mortality was recorded and dead fish were collected and frozen at -70 °C on daily basis. Sampling of ten fish from each tank was performed at ten days post challenge, and after this first sampling, ten fish were sampled from each tank once a month, up until six months post challenge.

Virus re-isolation from persistently infected fish

Fry samples stored at -70 °C without conservatives were added phosphate buffered saline (PBS) (1:5, weight/volume) and homogenized using a stomacher. 100μl of this homogenate was transferred to 600μl RLT buffer containing 2-mercaptoethanol (RNeasy Mini kit, Qiagen) and stored at -70 °C. The rest of the homogenate was diluted 1:2 in L-15 medium supplemented with 2 mM L-glutamine and 50μg $ml^{-1}$ gentamicin. After a brief centrifugation at 2500 x g for 10 minutes the supernatant were inoculated onto RTG-2 cells grown in 24 well plates in final dilutions of 1% and 0.1 %, and incubated for one week at 15 °C. The cell culture medium from the first passage was used to infect new monolayers. The samples were considered negative when no CPE was observed after 1-week incubation of the second passage. RNA was isolated from the fish homogenate for all negative samples on cell culture using the RNeasy Mini kit (Qiagen) in accordance with the supplier's protocol, and RT-PCR was performed to amplify a 224-bp IPNV-specific DNA fragment, as described by Santi et al. (2004. Identification of putative motifs involved in the virulence of infectious pancreatic necrosis virus. Virology 322:31-40) with minor modifications. The PCR products were separated by agarose gel electrophoresis and visualized by staining with SYBR® Safe DNA gel stain (Invitrogen).

Sequencing

RNA was isolated from homogenate stored on RLT buffer containing 2-mercaptoethanol (RNeasy Mini kit, Qiagen) stored at -70 °C after homogensation. RT-PCR was performed to amplify a 400-bp IPNV-specific DNA fragment using Qiagen's OneStep RT-PCR kit according to the manufacturer's instructions, with 0,5 μg RNA and 15 pmol each of primer A-Sp500F and A-Sp1689R (2004. Identification of putative motifs involved in the virulence of infectious pancreatic necrosis virus. Virology 322:31-40) in a total reaction volume of 25 μl. The cycling conditions were 50 °C for 30 min., 95 °C for 15 min., followed by 40 cycles at 94 °C for 45 s, 57 °C for 45s, 72 °C for 2 min.15s, and finally 72 °C for 10 min. The PCR products were separated by agarose gel electrophoresis and analyzed by staining with SYBR® Safe DNA gel stain.

To purify the DNA fragments from agarose gel, the Quantum Prep Freeze N' Squeeze DNA Gel Extraction Spin Column (BIO-RAD) was used according to the manufacturer's instructions. The recovered DNA was sequenced by a commercial sequencing service (Eurofins MWG operon) using primer A-Sp500F (2004. Identification of putative motifs involved in the virulence of infectious pancreatic necrosis virus. Virology 322:31-40). The sequence data were analyzed using VectorNTI software (Invitrogen).

Cloning and nucleotide sequencing

Four samples with different mixtures of bases in position 217 and 221 were picked for cloning and sequencing. *Taq* polymerase-amplified PCR products from the sequencing step were cloned into a TOPO® Vector by using TOPO TA Cloning® according to the manufacturers' instructions (Invitrogen). The One Shot® Chemically Competent *E. coli* cells were transformed according to the protocol and 50 μl were spread on prewarmed agar plates containing 50 μg/ml

ampicillin and pre-incubated for 30 min at 37 °C with 40 μl 40 mg/ml X-gal (Invitrogen) in dimethylformamide for white blue selection. 80-96 white colonies were picked from each Petri dish and placed one in each well on a 96 well agar plate with 150 mg ampicillin (GATC Biotech). Plasmid were isolated and sequenced by a commercial sequencing company, GATC Biotech. The sequence data were analyzed using VectorNTI software.

Results

Construction of low virulent IPNV Sp strain viruses

The isolate used for challenge had the following amino acid combinations in position 217, 221, and 247; ProThrAla. Amino acids in position 252, 281, 282 and 319 were ValThrAsnAla.

In detail, constructs including combinations of original constructs pUC19NVI15VP2 and pUC19NVI15B resulted in the recovery of NVI-15PA (2005. Molecular determinants of infectious pancreatic necrosis virus virulence and cell culture adaptation. Journal of Virology 79:10289-10299) which was subsequently passaged 10 times resulting in a mutation in position 221 in VP2 (Ala221Thr), and after plaque purification the rNVI-15PT isolate was recovered with the ProThrAla amino acid combination.

The genomic RNAs of the recovered viruses were analyzed after RT-PCR amplification, and the sequence analysis of the RT-PCR products confirmed the expected mutations in the VP2 and VP1 regions. Furthermore, complete nucleotide sequences of segment A of both viruses were determined, which did not exhibit any other unwanted nucleotide substitutions.

Persistent infection of fry

The challenge dose used in this study was lower than what is used in standard challenge studies (2004. Identification of putative motifs involved in the virulence of infectious pancreatic necrosis virus. Virology 322:31-40), due to establish a persistent infection in fry and to retain a high number of surviving fish (>95%). Of all the sampled fish during the experiment 94 % were persistently infected.

Reisolation and characterization of IPNV from infected fry

In order to document that the fish were infected and remained persistently infected we collected fry once every 30 days from month 1-6 post infection. At each time point 10 fish were examined using cell culture and RT-PCR. Overall IPNV were reisolated by culture or detected by RT-PCR in 94 % of the three groups. All fish were positive by 6 months post challenge, either by virus reisolation or RT-PCR.

Progression from acute infection to persistence has been associated with increase in genome complexity of hypervariable regions of viruses, particularly documented for HCV (2000. The outcome of acute hepatitis C predicted by the evolution of the viral quasispecies. Science 288:339-344. doi:8445 [pii]) and result in development of quasispecies. With the purpose to determine complexity of the virus genomes of the hypervariable region of the VP2 protein (amino acid positions 180-360) and any mutations in individual fish over the persistence period, RNA isolated from each of 5 fish from each group at all sampling points was amplified by RT-PCR and PCR-products were purified by gel electrophoresis and sent for sequencing. This showed that the ProThrAla strain had mutations in the genome yielding a combination of a ProThrAla (the challenge isolate) and a new ThrAlaThr variant, representing a high virulent, reverted strain of IPNV (Figure 5).

## Claims

**1.** A live avirulent infectious pancreatic necrosis virus (IPNV) which does not revert to a virulent virus after at least 3 passages in hosts known to be susceptible to IPNV; said virus comprising a nucleic acid encoding a mature VP2 protein or a precursor VP2 protein comprising an amino acid sequence having at least 85% sequence identity with SEQ ID NO. 1, wherein amino acid residues in position 252, 281, 282 and 319 of the protein are Asn, Ser, Asp and Glu respectively.

2. A live avirulent IPNV according to claim 1, which when delivered by immersion at a titre of 2 x $10^5$ $TCID_{50}$/ml to Atlantic salmon fry held in fresh water at a temperature of 12 °C causes the fry to be virus positive measured by reisolation on RTG-2 cells.

3. A live avirulent IPNV according to claim 1, which when delivered by immersion at a titre of 2 x $10^5$ $TCID_{50}$/ml to Atlantic salmon fry held in fresh water at a temperature of 12 °C provides the fry with protection against IPN disease as compared to non-infected fry.

4. A live avirulent IPNV according to claim 1, which when delivered by immersion at a titre of 2 x $10^5$ $TCID_{50}$/ml to Atlantic salmon fry held in fresh water at a temperature of 12 °C does not cause the fry to develop any signs of IPN disease.

5. A live avirulent IPNV according to claim 1, wherein the amino acid residue in position 221 of the protein is Thr.

6. A live avirulent IPNV according to claim 1 or 5, wherein the amino acid residue in position 217 of the protein is Pro.

7. A live avirulent IPNV according to claim 1, designated as IPNV-G700 and deposited under ECACC-No. 11 041201.

8. A live avirulent IPNV according to claim 1, which when delivered by immersion at a titre of 2 x $10^5$ $TCID_{50}$/ml to Atlantic salmon fry held in fresh water at a temperature of 12 °C

- causes the fry to be virus positive measured by reisolation on RTG-2 cells;
- causes the fry to be virus negative measured by immunohistochemistry;
- provides the fry with protection against IPN disease as compared to non-infected fry; and
- does not cause the fry to develop any signs of IPN disease.

9. Vaccine, comprising the live avirulent IPNV according to claim 1.

10. A live avirulent IPNV according to claim 1, for use as a vaccine in the prophylaxis or treatment of IPN disease.

11. A live avirulent IPNV according to claim 1, for use as a vaccine in the prophylaxis or treatment of IPN disease in fry.

12. A live avirulent IPNV according to claim 1 for use as a vaccine in the prophylaxis or treatment of IPN disease, wherein distribution is by immersion or oral administration.

**Patentansprüche**

1. Avirulentes Lebendvirus der infektiösen Pankreasnekrose (infectious pancreatic necrosis virus, IPNV), das nach mindestens 3 Passagen in Wirten, von denen bekannt ist, dass sie empfindlich gegenüber IPNV sind, nicht zu einem virulenten Virus zurückkehrt; wobei das Virus eine Nukleinsäure umfasst, die ein reifes VP2-Protein oder ein Vorläufer-VP2-Protein, umfassend eine Aminosäuresequenz, die eine mindestens 85%ige Sequenzidentität mit SEQ ID NO. 1 aufweist, kodiert, wobei Aminosäurereste in Position 252, 281, 282 und 319 des Proteins jeweils Asn, Ser, Asp und Glu sind.

2. Avirulentes Lebend-IPNV nach Anspruch 1, das bei Abgabe durch Immersion bei einem Titer von 2 x $10^5$ TCIDso/ml an Atlantiklachs-Brütlinge, die in Frischwasser bei einer Temperatur von 12°C gehalten werden, bewirkt, dass die Brütlinge viruspositiv sind, gemessen mittels Reisolierung auf RTG-2-Zellen.

3. Avirulentes Lebend-IPNV nach Anspruch 1, das bei Abgabe durch Immersion bei einem Titer von 2 x $10^5$ TCIDso/ml an Atlantiklachs-Brütlinge, die in Frischwasser bei einer Temperatur von 12°C gehalten werden, den Brütlingen einen Schutz gegen IPN-Erkrankung, verglichen mit nicht infizierten Brütlingen, bereitstellt.

4. Avirulentes Lebend-IPNV nach Anspruch 1, das bei Abgabe durch Immersion bei einem Titer von 2 x $10^5$ TCIDso/ml an Atlantiklachs-Brütlinge, die in Frischwasser bei einer Temperatur von 12°C gehalten werden, nicht bewirkt, dass die Brütlinge Anzeichen einer IPN-Erkrankung entwickeln.

5. Avirulentes Lebend-IPNV nach Anspruch 1, wobei der Aminosäurerest in Position 221 des Proteins Thr ist.

6. Avirulentes Lebend-IPNV nach Anspruch 1 oder 5, wobei der Aminosäurerest in Position 217 des Proteins Pro ist.

7. Avirulentes Lebend-IPNV nach Anspruch 1, das als IPNV-G700 bezeichnet wird und unter der ECACC-Nr. 11 041201 hinterlegt ist.

8. Avirulentes Lebend-IPNV nach Anspruch 1, das bei Abgabe durch Immersion bei einem Titer von 2 x $10^5$ TCIDso/ml an Atlantiklachs-Brütlinge, die in Frischwasser bei einer Temperatur von 12°C gehalten werden,

- bewirkt, dass die Brütlinge viruspositiv sind, gemessen mittels Reisolierung auf RTG-2-Zellen;
- bewirkt, dass die Brütlinge virusnegativ sind, gemessen mittels Immunhistochemie;
- den Brütlingen einen Schutz gegen IPN-Erkrankung, verglichen mit nicht infizierten Brütlingen, bereitstellt und
- nicht bewirkt, dass die Brütlinge Anzeichen einer IPN-Erkrankung entwickeln.

9. Impfstoff, umfassend das avirulente Lebend-IPNV nach Anspruch 1.

10. Avirulentes Lebend-IPNV nach Anspruch 1 zur Anwendung als Impfstoff bei der Prophylaxe oder Behandlung einer IPN-Erkrankung.

11. Avirulentes Lebend-IPNV nach Anspruch 1 zur Anwendung als Impfstoff bei der Prophylaxe oder Behandlung einer IPN-Erkrankung bei Brütlingen.

12. Avirulentes Lebend-IPNV nach Anspruch 1 zur Anwendung als Impfstoff bei der Prophylaxe oder Behandlung einer IPN-Erkrankung, wobei eine Verteilung durch Immersion oder orale Verabreichung erfolgt.

**Revendications**

1. Virus de la nécrose pancréatique infectieuse (VNPI) vivant avirulent qui ne redevient pas un virus virulent après au moins 3 passages chez des hôtes connus pour être sensibles au VNPI ; ledit virus comprenant un acide nucléique qui code une protéine VP2 mature ou un précurseur de la protéine VP2 comprenant une séquence d'acides aminés qui possède une identité de séquence d'au moins 85% avec la SEQ ID N° 1, les résidus acides aminés dans les positions 252, 281, 282 et 319 de la protéine étant Asn, Ser, Asp et Glu, respectivement.

2. VNPI vivant avirulent selon la revendication 1 qui, lorsqu'il est administré par immersion à un titre de 2 x $10^5$ $DICT_{50}$/ml à des alevins de saumon atlantique maintenus dans de l'eau douce à une température de 12 °C, rend les alevins positifs pour le virus, ainsi que mesuré par réisolation sur des cellules RTG-2.

3. VNPI vivant avirulent selon la revendication 1 qui, lorsqu'il est administré par immersion à un titre de 2 x $10^5$ $DICT_{50}$/ml à des alevins de saumon atlantique maintenus dans de l'eau douce à une température de 12 °C, confère aux alevins une protection contre la maladie NPI, comparés à des alevins non infectés.

4. VNPI vivant avirulent selon la revendication 1 qui, lorsqu'il est administré par immersion à un titre de 2 x $10^5$ $DICT_{50}$/ml à des alevins de saumon atlantique maintenus dans de l'eau douce à une température de 12 °C, ne provoque pas chez les alevins le développement de signes de la maladie NPI.

5. VNPI vivant avirulent selon la revendication 1, dans lequel le résidu acide aminé en position 221 de la protéine est Thr.

6. VNPI vivant avirulent selon la revendication 1 ou la revendication 5, dans lequel le résidu acide aminé en position 217 de la protéine est Pro.

7. VNPI vivant avirulent selon la revendication 1, désigné VNPI-G700 et déposé sous le numéro ECACC 11 041201.

8. VNPI vivant avirulent selon la revendication 1 qui, lorsqu'il est administré par immersion à un titre de 2 x $10^5$ $DICT_{50}$/ml à des alevins de saumon atlantique maintenus dans de l'eau douce à une température de 12 °C,

- rend les alevins positifs pour le virus, ainsi que mesuré par réisolation sur des cellules RTG-2 ;
- rend les alevins négatifs pour le virus, ainsi que mesuré par immunohistochimie ;
- confère aux alevins une protection contre la maladie NPI, comparés à des alevins non infectés ; et

- ne provoque pas chez les alevins le développement de signes de la maladie NPI.

**9.** Vaccin comprenant le VNPI vivant avirulent selon la revendication 1.

**10.** VNPI vivant avirulent selon la revendication 1, destiné à être utilisé comme vaccin dans la prévention ou le traitement de la maladie NPI.

**11.** VNPI vivant avirulent selon la revendication 1, destiné à être utilisé comme vaccin dans la prévention ou le traitement de la maladie NPI chez des alevins.

**12.** VNPI vivant avirulent selon la revendication 1, destiné à être utilisé comme vaccin dans la prévention ou le traitement de la maladie NPI, la distribution se faisant par immersion ou par administration orale.

Figure 1

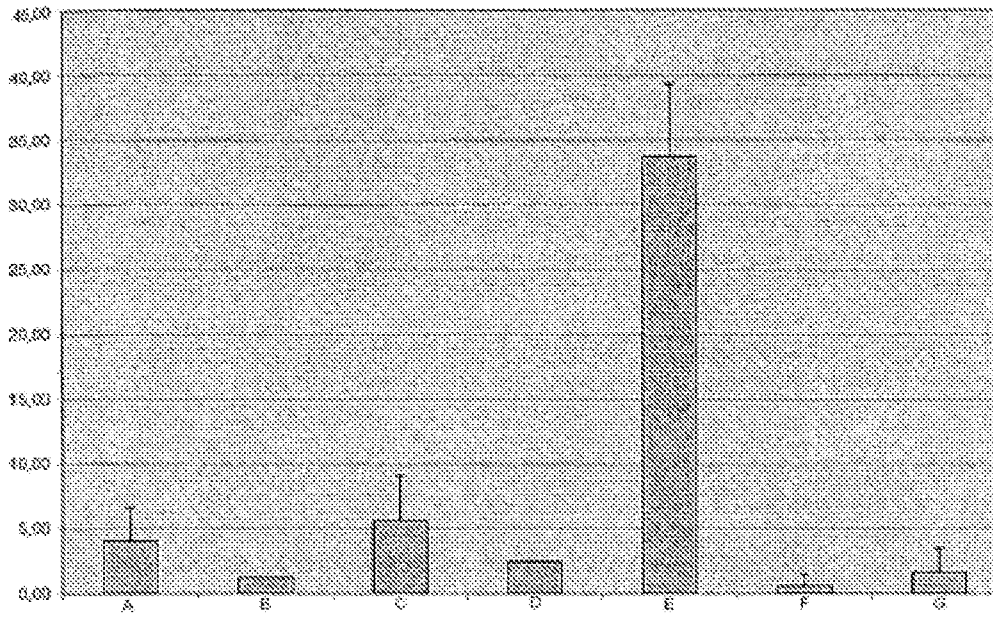


Figure 2

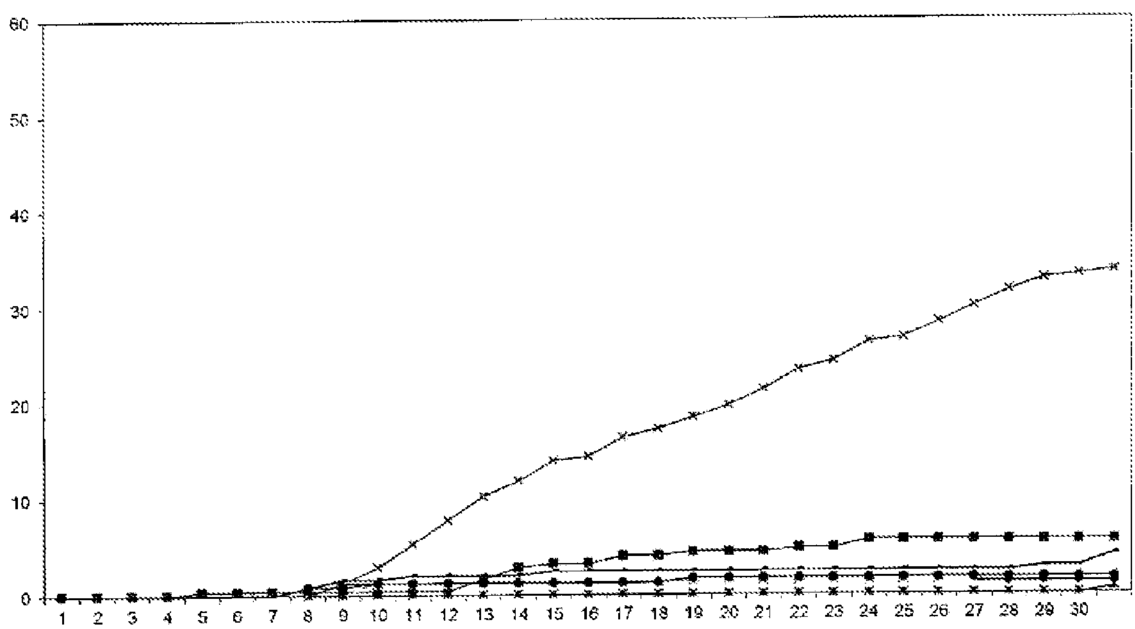

Figure 3

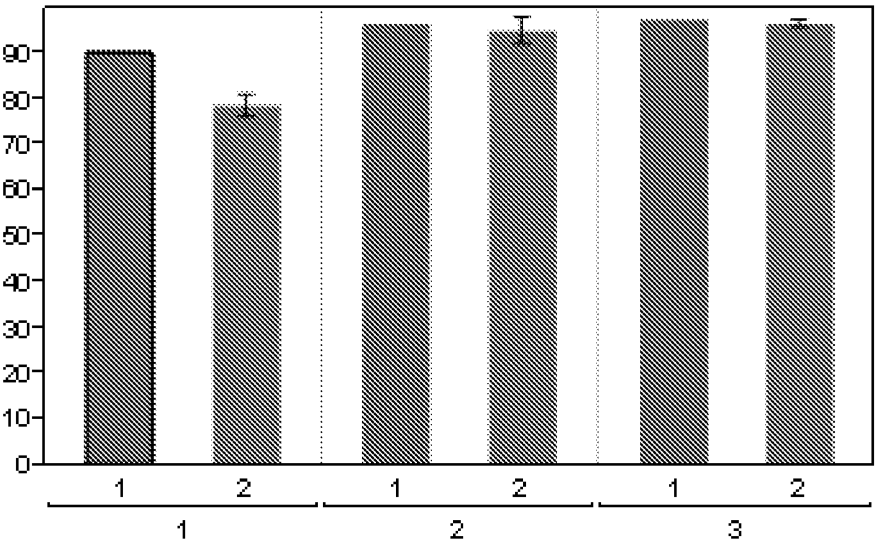


Figure 4

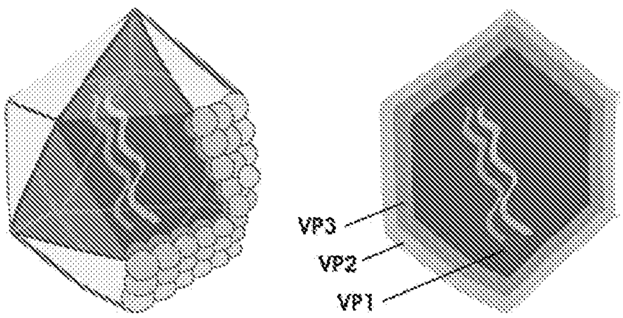


Figure 5

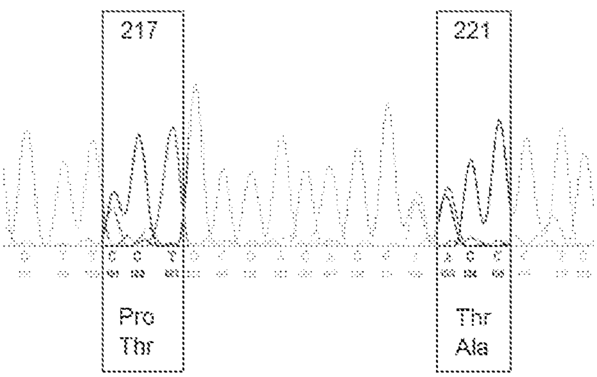

**Figure 6a**

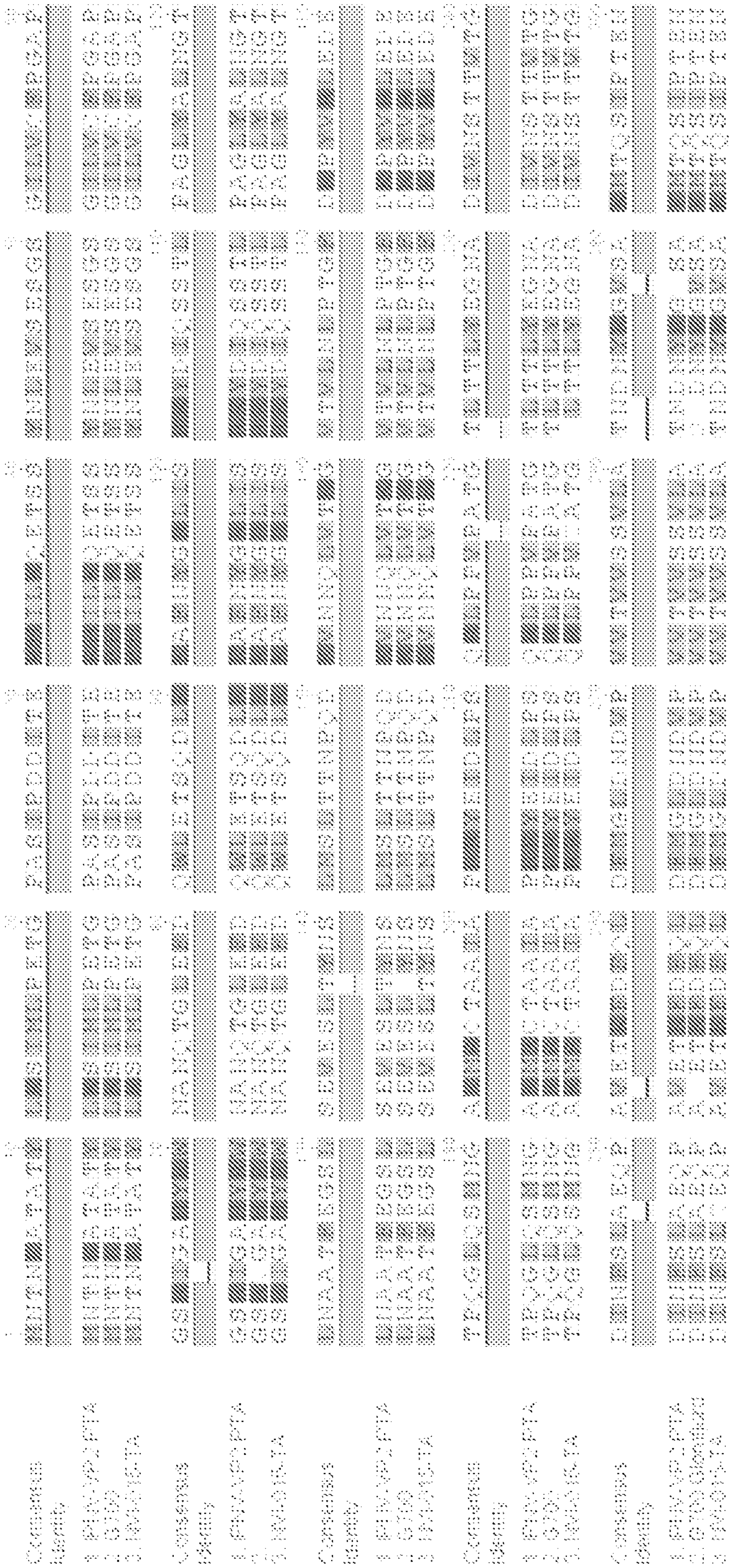

**Figure 6b**

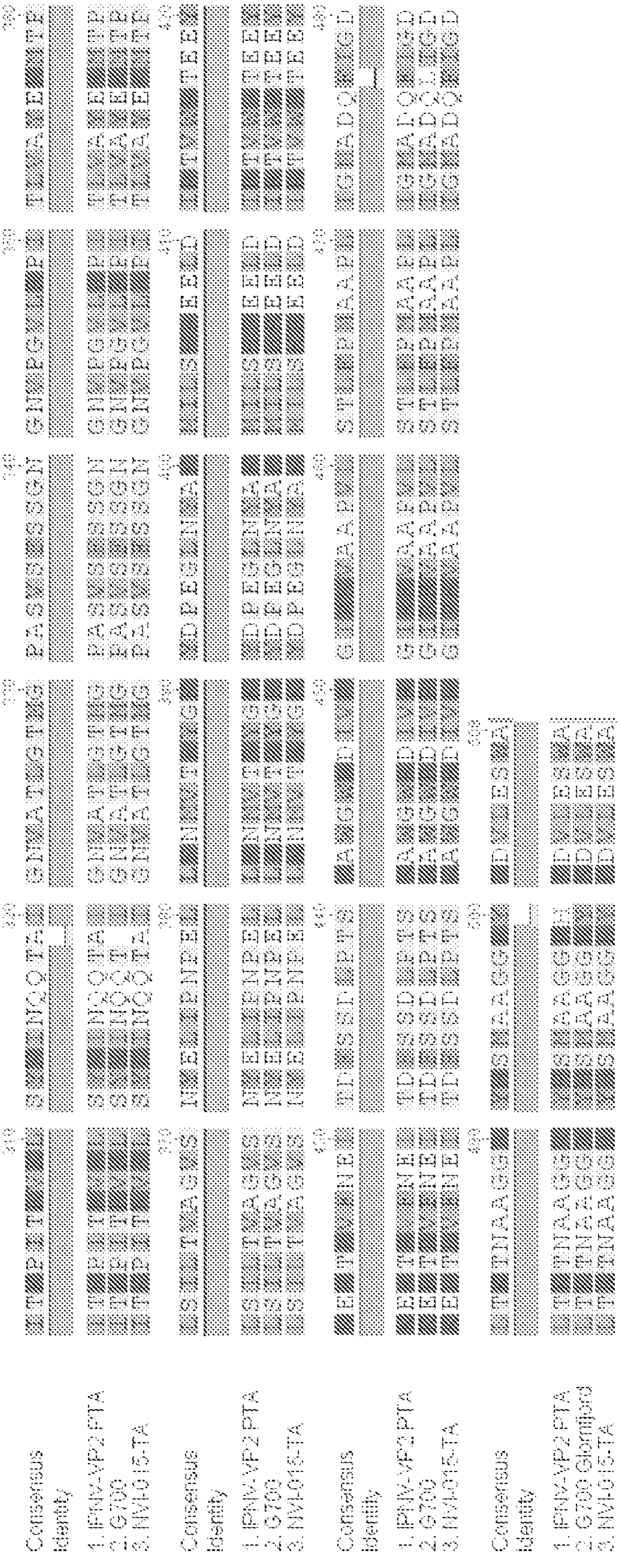

## Figure 7a

ONE AND THREE LETTER SYMBOLS FOR THE AMINO ACIDS[a]

| | | | | | |
|---|---|---|---|---|---|
| A | Ala | Alanine | M | Met | Methionine |
| B | Asx | Asparagine or aspartic acid | N | Asn | Asparagine |
| C | Cys | Cysteine | P | Pro | Proline |
| D | Asp | Aspartic acid | Q | Gln | Glutamine |
| E | Glu | Glutamic acid | R | Arg | Arginine |
| F | Phe | Phenylalanine | S | Ser | Serine |
| G | Gly | Glycine | T | Thr | Threonine |
| H | His | Histidine | V | Val | Valine |
| I | Ile | Isoleucine | W | Trp | Tryptophan |
| K | Lys | Lysine | Y | Tyr | Tyrosine |
| L | Leu | Leucine | Z | Glx | Glutamine or glutamic acid |

[a] The one letter symbol for an undetermined or nonstandard amino acid is X.

## Figure 7b

THE STANDARD GENETIC CODE

| First Position (5′ end) | Second Position | | | | Third Position (3′ end) |
|---|---|---|---|---|---|
| | U | C | A | G | |
| U | UUU Phe | UCU Ser | UAU Tyr | UGU Cys | U |
| | UUC Phe | UCC Ser | UAC Tyr | UGC Cys | C |
| | UUA Leu | UCA Ser | UAA Stop | UGA Stop | A |
| | UUG Leu | UCG Ser | UAG Stop | UGG Trp | G |
| C | CUU Leu | CCU Pro | CAU His | CGU Arg | U |
| | CUC Leu | CCC Pro | CAC His | CGC Arg | C |
| | CUA Leu | CCA Pro | CAA Gln | CGA Arg | A |
| | CUG Leu | CCG Pro | CAG Gln | CGG Arg | G |
| A | AUU Ile | ACU Thr | AAU Asn | AGU Ser | U |
| | AUC Ile | ACC Thr | AAC Asn | AGC Ser | C |
| | AUA Ile | ACA Thr | AAA Lys | AGA Arg | A |
| | AUG Met[a] | ACG Thr | AAG Lys | AGG Arg | G |
| G | GUU Val | GCU Ala | GAU Asp | GGU Gly | U |
| | GUC Val | GCC Ala | GAC Asp | GGC Gly | C |
| | GUA Val | GCA Ala | GAA Glu | GGA Gly | A |
| | GUG Val | GCG Ala | GAG Glu | GGG Gly | G |

[a] AUG forms part of the initiation signal as well as coding for internal Met residues.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9950419 A
- NO 20110650
- NO 20110402

### Non-patent literature cited in the description


- **ORPETVEIT et al.** *Molecular epidemiology of infectious pancreatic necrosis virus (IPNV) in Norway*
- **KÄRBER G.** *Beitrag zur kollektiven Behandlung pharmakologischer Reihenversueche,* 1931, vol. 162
- **KARBER.** Beitrage zur kollektiven Behandlung pharmakologischer Reihenversuche. *Arch.Exp.Pathol.Pharmakol,* 1931, vol. 162, 480-483
- **YAO ; VAKHARIA.** Generation of infectious pancreatic necrosis virus from cloned cDNA. *J Virol.,* 1998, vol. 72, 8913-8920
- **SONG.** Molecular determinants of infectious pancreatic necrosis virus virulence and cell culture adaptation. *Journal of Virology,* 2005, vol. 79, 10289-10299
- Molecular determinants of infectious pancreatic necrosis virus virulence and cell culture adaptation. *Journal of Virology,* 2005, vol. 79, 10289-10299
- Infectious pancreatic necrosis virus induces apoptosis in vitro and in vivo independent of VP5 expression. *Virology,* 2005, vol. 342, 13-25
- Identification of putative motifs involved in the virulence of infectious pancreatic necrosis virus. *Virology,* vol. 322, 31-40
- **YAO ; VAKHARIA.** Generation of infectious pancreatic necrosis virus from cloned cDNA. *J Virol,* 1998, vol. 72, 8913-8920
- Molecular determinants of infectious pancreatic necrosis virus virulence and cell culture adaptation. *Journal of Virology,* 2005, vol. 79, 10289-10299
- **SANTI et al.** Identification of putative motifs involved in the virulence of infectious pancreatic necrosis virus. *Virology,* 2004, vol. 322, 31-40
- Identification of putative motifs involved in the virulence of infectious pancreatic necrosis virus. *Virology,* 2004, vol. 322, 31-40
- Identification of putative motifs involved in the virulence of infectious pancreatic necrosis virus. *Virology,* 2004, vol. 322, 31-40
- The outcome of acute hepatitis C predicted by the evolution of the viral quasispecies. *Science,* 2000, vol. 288, 339-344

SEQUENCE LISTING

<110> Norwegian School of Veterinary Science

<120> IPN vaccine

<130> P61003001PCT00

<150> NO20110650
<151> 2011-05-02

<150> NO20110402

<151> 2011-03-16

<160> 3

<170> PatentIn version 3.5

<210> 1
<211> 508
<212> PRT
<213> Infectious pancreatic necrosis virus

<220>
<221> PROPEP
<222> (1)..(508)
<223> Precursor VP2

<220>
<221> mat_peptide
<222> (1)..(442)
<223> Mature VP2

<400> 1

```
Met Asn Thr Asn Lys Ala Thr Ala Thr Tyr Leu Lys Ser Ile Met Leu
1               5                   10                  15

Pro Glu Thr Gly Pro Ala Ser Ile Pro Asp Asp Thr Thr Glu Arg His
            20                  25                  30

Ile Leu Lys Gln Glu Thr Ser Ser Tyr Asn Leu Glu Val Ser Glu Ser
        35                  40                  45

Gly Ser Gly Ile Leu Val Cys Phe Pro Gly Ala Pro Gly Ser Arg Val
    50                  55                  60

Gly Ala His Tyr Arg Trp Asn Ala Asn Gln Thr Gly Leu Glu Phe Asp
65                  70                  75                  80

Gln Trp Leu Glu Thr Ser Gln Asp Leu Lys Lys Ala Phe Asn Tyr Gly
                85                  90                  95

Arg Leu Ile Ser Arg Lys Tyr Asp Ile Gln Ser Ser Thr Leu Pro Ala
            100                 105                 110
```

Gly Leu Tyr Ala Leu Asn Gly Thr Leu Asn Ala Ala Thr Phe Glu Gly
115 120 125

Ser Leu Ser Glu Val Glu Ser Leu Ala Tyr Asn Ser Leu Met Ser Leu
130 135 140

Thr Thr Asn Pro Gln Asp Lys Val Asn Asn Gln Leu Val Thr Lys Gly
145 150 155 160

Val Thr Val Leu Asn Leu Pro Thr Gly Phe Asp Lys Pro Tyr Val Arg
165 170 175

Leu Glu Asp Glu Thr Pro Gln Gly Ile Gln Ser Met Asn Gly Ala Lys
180 185 190

Met Arg Cys Thr Ala Ala Ile Ala Pro Arg Arg Tyr Glu Ile Asp Leu
195 200 205

Pro Ser Gln Arg Leu Pro Pro Val Pro Ala Thr Gly Thr Leu Thr Thr
210 215 220

Leu Tyr Glu Gly Asn Ala Asp Ile Val Asn Ser Thr Thr Val Thr Gly
225 230 235 240

Asp Ile Asn Phe Ser Leu Ala Glu Gln Pro Ala Asn Glu Thr Lys Phe
245 250 255

Asp Phe Gln Leu Asp Phe Met Gly Leu Asp Asn Asp Val Pro Val Val
260 265 270

Thr Val Val Ser Ser Val Leu Ala Ser Asp Asp Asn Tyr Arg Gly Val
275 280 285

Ser Ala Lys Met Thr Gln Ser Ile Pro Thr Glu Asn Ile Thr Lys Pro
290 295 300

Ile Thr Arg Val Lys Leu Ser Tyr Lys Ile Asn Gln Gln Thr Glu Ile
305 310 315 320

Gly Asn Val Ala Thr Leu Gly Thr Met Gly Pro Ala Ser Val Ser Phe
325 330 335

Ser Ser Gly Asn Gly Asn Val Pro Gly Val Leu Arg Pro Ile Thr Leu
340 345 350

Val Ala Tyr Glu Lys Met Thr Pro Leu Ser Ile Leu Thr Val Ala Gly
355 360 365

Val Ser Asn Tyr Glu Leu Ile Pro Asn Pro Glu Leu Leu Lys Asn Met
370 375 380

Val Thr Arg Tyr Gly Lys Tyr Asp Pro Glu Gly Leu Asn Tyr Ala Lys
385 390 395 400

Met Ile Leu Ser His Arg Glu Glu Leu Asp Ile Arg Thr Val Trp Arg
405 410 415

Thr Glu Glu Tyr Lys Glu Arg Thr Arg Val Phe Asn Glu Ile Thr Asp
420 425 430

Phe Ser Ser Asp Leu Pro Thr Ser Lys Ala Trp Gly Trp Arg Asp Ile
435 440 445

Val Arg Gly Ile Arg Lys Val Ala Ala Pro Val Leu Ser Thr Leu Phe
450 455 460

Pro Met Ala Ala Pro Leu Ile Gly Met Ala Asp Gln Phe Ile Gly Asp
465 470 475 480

Leu Thr Lys Thr Asn Ala Ala Gly Gly Arg Tyr His Ser Met Ala Ala
485 490 495

Gly Gly Arg Tyr Lys Asp Val Leu Glu Ser Trp Ala
500 505

<210> 2
<211> 1524
<212> DNA
<213> Infectious pancreatic necrosis virus

<400> 2

atgaacacaa acaaggcaac cgcgacctac ctgaaatcca ttatgcttcc agagactgga 60

ccagcaagca tcccggacga cataacggag agacacatct taaaacaaga gacctcgtca 120

tacaacctag aggtctccga atcaggaagt ggcattcttg tttgtttccc tggggcacca 180

ggctcacggg tcggtgcaca ctacagatgg aatgcgaacc agacggggct ggagttcgac 240

cagtggctgg agacgtcgca ggacctgaag aaagccttca attacgggag gctgatctca 300

aggaaatacg acatccaaag ctccacacta ccggccggtc tctacgctct gaacgggacg 360

ctcaacgctg ccaccttcga gggcagtctg tccgaggtgg agagcctggc ctacaacagc 420

ctgatgtccc taacaacgaa cccccaggac aaagtcaaca accagctggt gacaaaagga 480

gtcacagtcc tgaatctacc aacagggttc gacaaaccat acgtccgcct agaggacgag 540

acacccccagg gtctccagtc aatgaacggg gccaagatga ggtgcacagc tgcaattgca 600

```
ccgcggaggt acgagatcga cctcccatcc caacgcctac cccccgttcc tgcgacagga      660
accctcacca ctctctacga gggaaacgcc gacatcgtca actccacaac agtgacggga      720
gacataaact tcagtctggc agaacaaccc gcaaacgaga ccaagttcga cttccagctg      780
gacttcatgg gccttgacaa cgacgtccca gttgtcacag tggtcagctc cgtgctggcc      840
tcagatgaca actacagagg agtctcagcc aagatgaccc agtccatccc gaccgagaac      900
atcacaaagc cgatcaccag ggtcaagctg tcatacaaga tcaaccagca gacagaaatc      960
ggcaacgtcg ccaccctggg cacaatgggt ccagcatccg tctccttctc atcagggaac     1020
ggaaatgtcc ccggcgtgct cagaccaatc acactggtgg cctatgagaa gatgacaccg     1080
ctgtccatcc tgaccgtagc tggagtgtcc aactacgagc tgatcccaaa cccagaactc     1140
ctcaagaaca tggtgacacg ctatggcaag tacgaccccg aaggtctcaa ctatgccaag     1200
atgatcctgt cccacaggga agagctggac atcaggacag tgtggaggac agaggagtac     1260
aaggagagga ccagagtctt caacgagatc acggacttct ccagtgacct gcccacgtca     1320
aaggcatggg gctggagaga catagtcaga ggaattcgga aagtcgcagc tcctgtactg     1380
tccacgctgt ttccaatggc agcaccactc ataggaatgg cagaccaact cattggagat     1440
ctcactaaga ccaatgcagc aggcggaagg taccactcca tggccgcagg agggcgctac     1500
aaagacgtgc tcgagtcctg ggca                                            1524
```

<210> 3
<211> 1524
<212> RNA
<213> Infectious pancreatic necrosis virus

<400> 3

```
augaacacaa acaaggcaac cgcgaccuac cugaaaucca uuaugcuucc agagacugga     60
ccagcaagca ucccggacga cauaacggag agacacaucu uaaaacaaga gaccucguca    120
uacaaccuag aggucuccga aucaggaagu ggcauucuug uuuguuuccc uggggcacca    180
ggcucacggg ucggugcaca cuacagaugg aaugcgaacc agacggggcu ggaguucgac    240
caguggcugg agacgucgca ggaccugaag aaagccuuca auuacgggag gcugaucuca    300
aggaaauacg acauccaaag cuccacacua ccggccgguc ucuacgcucu gaacgggacg    360
cucaacgcug ccaccuucga gggcagucug uccgaggugg agagccuggc cuacaacagc    420
cugaugucccc uaacaacgaa cccccaggac aaagucaaca accagcuggu gacaaaagga    480
gucacagucc ugaaucuacc aacaggguuc gacaaaccau acguccgccu agaggacgag    540
acaccccagg gucuccaguc aaugaacggg gccaagauga ggugcacagc ugcaauugca    600
ccgcggaggu acgagaucga ccucccaucc caacgccuac cccccguucc ugcgacagga    660
acccucacca cucucuacga gggaaacgcc gacaucguca acuccacaac agugacggga    720
gacauaaacu ucagucuggc agaacaaccc gcaaacgaga ccaaguucga cuuccagcug    780
gacuucaugg gccuugacaa cgacguccca guugucacag uggucagcuc cgugcuggcc    840
ucagaugaca acuacagagg agucucagcc aagaugaccc aguccauccc gaccgagaac    900
aucacaaagc cgaucaccag ggucaagcug ucauacaaga ucaaccagca gacagaaauc    960
ggcaacgucg ccacccuggg cacaaugggu ccagcauccg ucuccuucuc aucagggaac   1020
ggaaaugucc ccggcgugcu cagaccaauc acacuggugg ccuaugagaa gaugacaccg   1080
cuguccaucc ugaccguagc uggaguguccc aacuacgagc ugaucccaaa cccagaacuc   1140
cucaagaaca uggugacacg cuauggcaag uacgaccccg aaggucucaa cuaugccaag   1200
augauccugu cccacaggga agagcuggac aucaggacag uguggaggac agaggaguac   1260
aaggagagga ccagagucuu caacgagauc acggacuucu ccagugaccu gcccacguca   1320
aaggcauggg gcuggagaga cauagucaga ggaauucgga aagucgcagc uccuguacug   1380
uccacgcugu uuccaauggc agcaccacuc auaggaaugg cagaccaacu cauuggagau   1440
cucacuaaga ccaaugcagc aggcggaagg uaccacucca uggccgcagg agggcgcuac   1500
aaagacgugc ucgaguccug ggca                                          1524
```